# EUROPEAN PATENT APPLICATION

(11) **EP 4 700 126 A2**
(43) Date of publication of application: **25.02.2026**
(21) Application number: 25214109.8
(22) Date of filing: 23.07.2021
(51) Int. Cl.: C12N 15/86

(54) **OPTIMIZED SLC13A5 GENES AND EXPRESSION CASSETTES AND THEIR USE**

(30) Priority: 23.07.2020 US 202063055795 P
(62) Divisional of application: 21845857.8
(71) Applicant: The University of North Carolina at Chapel Hill, Chapel Hill, NC 27514 (US)
(72) Inventor: BAILEY, Rachel, Trophy Club, Texas 76262 (US); GRAY, Steven James, Southlake, Texas 76092 (US)
(74) Representative: HGF

(57) **Abstract**

This invention relates to polynucleotides comprising optimized SLC13A5 open reading frame (ORF) sequences, vectors comprising the same, and methods of using the same for delivery of the ORF to a cell or a subject and to treat disorders associated with aberrant expression of a SLC13A5 gene or aberrant activity of a SLC13A5 gene product in the subject, such as citrate transporter disorder.

## Description

### STATEMENT OF PRIORITY

This application claims the benefit, under 35 U.S.C. § 119(e), of U.S. Provisional Application No. 63/055,795, filed on July 23, 2020, the entire contents of which are incorporated by reference herein.

### STATEMENT REGARDING ELECTRONIC FILING OF A SEQUENCE LISTING

A Sequence Listing in ASCII text format, submitted under 37 C.F.R. § 1.821, entitled 5470-883WO_ST25.txt, 8,769 bytes in size, generated on July 12, 2021 and filed via EFS-Web, is provided in lieu of a paper copy. This Sequence Listing is hereby incorporated herein by reference into the specification for its disclosures.

### FIELD OF THE INVENTION

This invention relates to polynucleotides comprising optimized SLC13A5 open reading frame (ORF) sequences, vectors comprising the same, and methods of using the same for delivery of the ORF to a cell or a subject and to treat disorders associated with aberrant expression of a SLC13A5 gene or aberrant activity of a SLC13A5 gene product in the subject, such as citrate transporter disorder.

### BACKGROUND OF THE INVENTION

Solute carrier family 13 (sodium-dependent citrate transporter) member 5, also known as the Na⁺/citrate cotransporter, is a protein that in humans is encoded by the SLC13A5 gene. SLC13A5 is a tricarboxylate plasma transporter with a preference for citrate. Genetic mutations of the SLC13A5 gene are the cause of an extremely rare citrate transporter disorder, also known as SLC13A5 deficiency. Mutations in SLC13A5 cause autosomal recessive epileptic encephalopathy with seizure onset in the first days of life. Those afflicted suffer from seizures, global developmental delay, movement disorder, and hypotonia.

Affected children present with seizures beginning within a few days of birth which persist throughout life. They show difficulty with speech production, limited and slow motor progress with problems standing or walking independently. Problems with tone are also reported with episodes of body stiffening and weakening. Almost all the affected children have abnormalities in their tooth enamel. Brain MRIs appear normal or have subtle changes in the white matter. Citrate levels in the blood of patients is mildly elevated.

SLC13A5 mutations described to date result in no or a reduced amount of citrate transporter activity. Citrate is a key metabolite and is known to play an important role in the energy generation pathways in the cells. In addition, it could also be involved in regulating the concentration of other ions inside and outside of the cells due to its chelating properties. Although SLC13A5 transporter is extensively studied in liver cells, the function of this transporter in the human brain is largely unknown.

At this time, there are no specific treatments that cure the disease. Treating seizures in children with citrate transporter disorder has proven difficult in some patients. Although anti-seizure medications have been successful in controlling seizures in some patients, most patients have limited success with the available drugs. Multiple seizure drugs including acetazolamide and ketogenic diet have been tried with mixed efficacy to manage symptoms of SLC13A5 deficiency. There is a need to provide a meaningful and long-term therapeutic benefit for this population.

### SUMMARY OF THE INVENTION

The present invention is based, in part, on the development of optimized SLC13A5 genes, expression cassettes, and vectors capable of providing therapeutic levels of SLC13A5 expression for treating disorders associated with SLC13A5 expression such as citrate transporter disorder.

Thus, one aspect of the invention relates to a polynucleotide comprising a human SLC13A5 open reading frame, wherein the human SLC13A5 open reading frame has been codon-optimized for expression in human cells.

A further aspect of the invention relates to an expression cassette comprising a polynucleotide comprising a human SLC13A5 open reading frame and vectors, transformed cells, and transgenic animals comprising the polynucleotide of the invention.

Another aspect of the invention relates to a pharmaceutical formulation comprising the polynucleotide, expression cassette, vector, and/or transformed cell of the invention in a pharmaceutically acceptable carrier.

An additional aspect of the invention relates to a method of expressing a SLC13A5 open reading frame in a cell, comprising contacting the cell with the polynucleotide, expression cassette, and/or vector of the invention, thereby expressing the SLC13A5 open reading frame in the cell.

A further aspect of the invention relates to a method of expressing a SLC13A5 open reading frame in a subject, comprising delivering to the subject the polynucleotide, expression cassette, vector, and/or transformed cell of the invention, thereby expressing the SLC13A5 open reading frame in the subject.

An additional aspect of the invention relates to a method of treating a disorder associated with aberrant expression of a SLC13A5 gene or aberrant activity of a SLC13A5 gene product in a subject in need thereof, comprising administering to the subject a therapeutically effective amount of the polynucleotide, expression cassette, vector, and/or transformed cell of the invention, such that the SLC13A5 open reading frame is expressed in the subject.

A further aspect of the invention relates to a method of treating citrate transporter disorder in a subject in need thereof, comprising administering to the subject a therapeutically effective amount of the polynucleotide, expression cassette, vector, and/or transformed cell of the invention, such that the SLC13A5 open reading frame is expressed in the subject.

Another aspect of the invention relates to a polynucleotide, expression cassette, vector, and/or transformed cell of the invention for use in a method of treating a disorder associated with aberrant expression of a SLC13A5 gene or aberrant activity of a SLC13A5 gene product in a subject in need thereof.

These and other aspects of the invention are set forth in more detail in the description of the invention below.

### BRIEF DESCRIPTION OF THE DRAWINGS

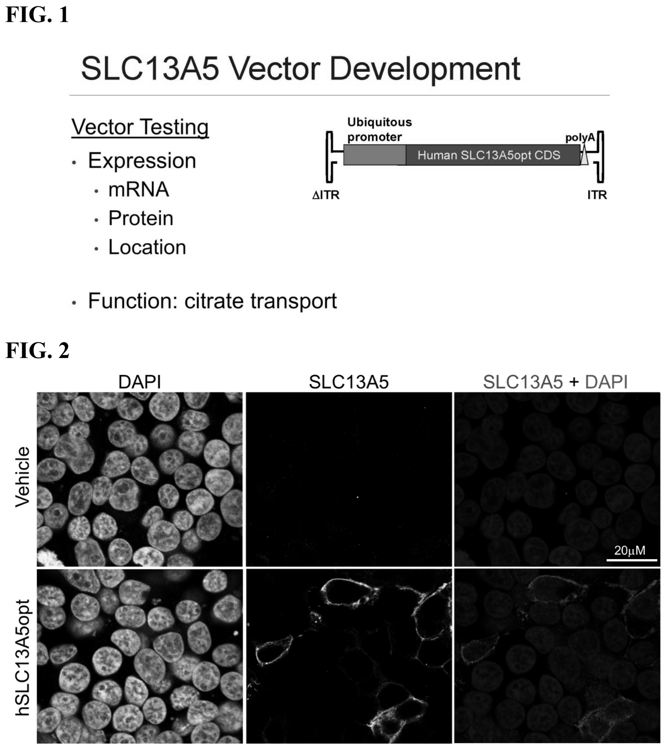

**FIG. 1** shows a schematic of the hSLC13A5opt construct design.
**FIG. 2** shows hSLC13A5opt protein expression in HEK293T cells. Confocal microscopy of HEK293T cells transfected with no vector (vehicle, top panel) or 0.5 µg of pUSP-hSLC13A5ₒₚₜ. 48 hours post-transfection stained with an antibody against human SLC13A5 showed no signal in vehicle treated cells while hSLC13A5opt transfected cells had a punctate pattern surrounding the cell. DNA was counterstained with DAPI. Left panel: DAPI; middle panel, anti-SLC13A5; right panel, merged image of anti-SLC13A5 and DAPI.

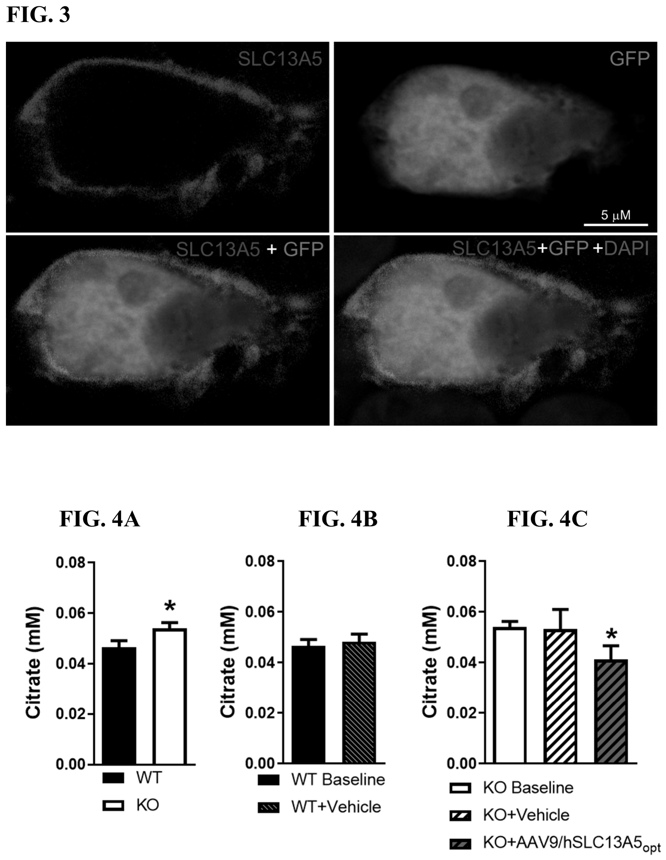

**FIG. 3** shows that hSLC13A5ₒₚₜ protein localizes to plasma membrane. Confocal microscopy of HEK293T cell transfected with 0.5µg pCbh-GFP and 0.5µg pUSP-hSLC13A5ₒₚₜ. 48 hours after transfection, GFP fluorescence (top right panel, alone; bottom panels, merged with SLC13A5 and DAPI) was detected homogenously in the cytosol and did not co-localize with SLC13A5 staining (shown alone in top left panel; bottom panels, merged with GFP and DAPI), which was in a punctate pattern in the plasma membrane. DNA was counterstained with DAPI.
**FIGS. 4A-4C** show that intrathecal AAV9/UsP-hSLC13A5opt treatment reduces plasma citrate levels in Slc13a5 KO mice. Plasma was isolated from Slc13a5 knock-out (KO) mice and wild-type (WT) littermates before and after intrathecal delivery of 8 x 10¹¹ vg scAAV9/hSLC13A5ₒₚₜ or vehicle as a control and citrate was quantified by GC/MS analysis. **FIG. 4A** shows that similarly to SLC13A5 patients, Slc13a5 KO mice (right bar) have significantly increased baseline plasma citrate levels compared to WT mice (left bar). 1 month post- injection, WT mice **(****FIG. 4B****)** and KO mice **(****FIG. 4C****)** treated with vehicle **(****FIG. 4B****,** right bar; **FIG. 4C****,** middle bar) had equivalent plasma citrate levels compared to their respective baseline plasma levels, while KO mice treated with scAAV9/hSLC13A5ₒₚₜ **(****FIG. 4C****,** right bar) have significantly decreased plasma citrate levels. Data presented as mean ± SEM. *p<0.05 unpaired student's t-test **(****FIG. 4A****)** or one-way analysis of variance (ANOVA) followed by Dunnett's multiple comparisons test **(****FIG. 4C****).** n=9 WT and 10 KO.

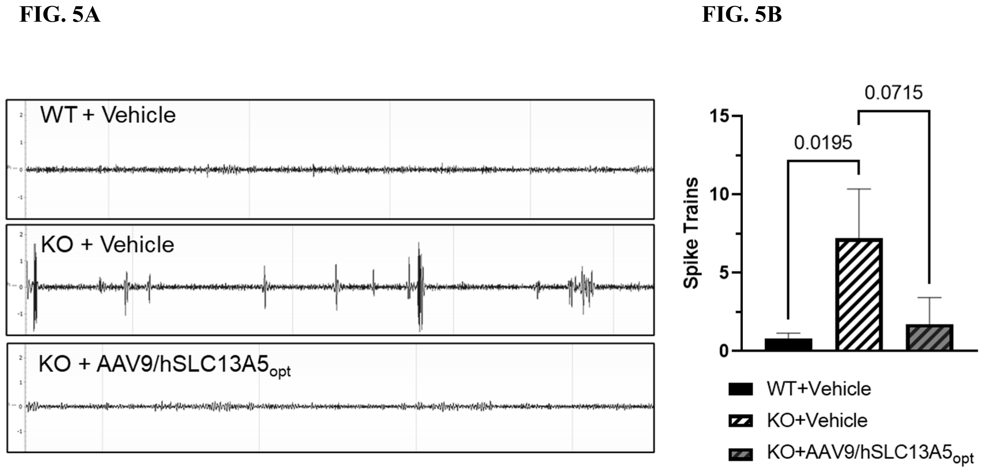

**FIGS. 5A-5B** show that intrathecal AAV9/UsP-hSLC13A5opt treatment reduces EEG activity to normal levels in Slc13a5 KO mice. Slc13a5 knock-out (KO) mice and wild-type (WT) littermates were treated with vehicle or 8 x 10¹¹ vg AAV9/UsP-hSLC13A5opt at ~ 3 months of age. At ~ 5 months post-injection, mice received wireless telemetry implants and EEG activity was recorded for two 60-hour sessions. **FIG. 5A** shows representative EEG tracings in WT and KO mice treated with vehicle or AAV9/UsP-hSLC13A5opt. Vehicle treated KO mice have increased epileptic discharges compared to WT mice or vector treated KO mice. **FIG. 5B** shows the quantification of epileptic spike trains. Vehicle treated KO mice **(****FIG. 5B****,** middle bar) have an increased number of spike trains as compared to vehicle treated WT mice **(****FIG. 5B****,** left bar) and AAV9/UsP-hSLC13A5opt treated KO mice **(FIG. 5C,** left bar). Data presented as mean ± SEM. One-way analysis of variance (ANOVA) with Fisher's post-hoc analysis was used to evaluate statistical difference. n=12 WT+Vehicle, n=9 KO+Vehicle, n=7 KO+AAV9/hSLC13A5opt.

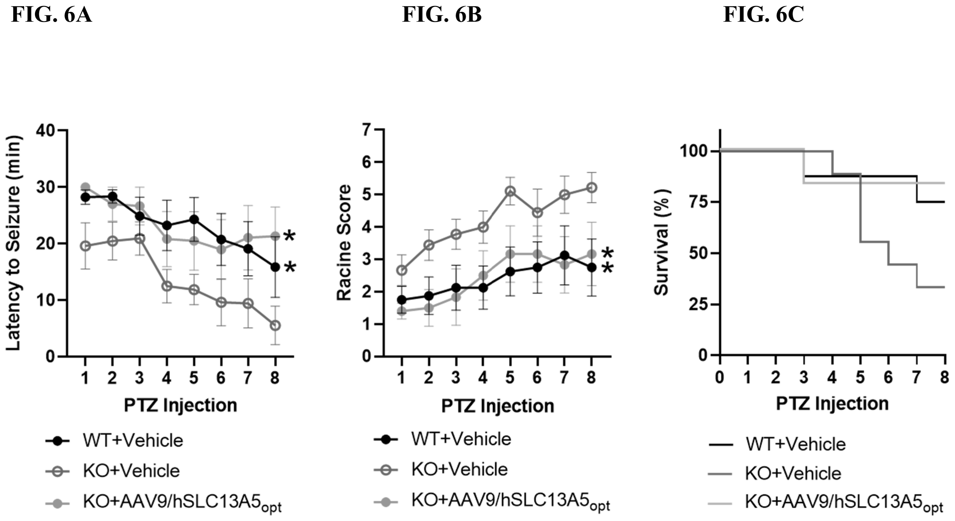

**FIG. 6A-6C** show that intrathecal AAV9/UsP-hSLC13A5opt treatment reduces seizure susceptibility and seizure-related death in Slc13a5 KO mice. Slc13a5 knock-out (KO) mice and wild-type (WT) littermates were treated with vehicle or AAV9/UsP-hSLC13A5opt at ~ 3 months of age. At ~ 6 months post-injection, following EEG recordings of baseline activity, mice received repetitive, low-dose (30 mg/kg) pentylenetetrazol (PTZ) injections and the latency to convulsive seizures **(****FIG. 6A****)** and severity of seizures **(****FIG. 6B****)** were measured using the Racine Scale. **FIG. 6A** show vehicle treated KO mice have significantly shorter latency to seizure onset compared to vehicle treated WT littermates. Treatment with AAV9/UsP-hSLC13A5opt fully reduced the seizure latency to normal levels. **FIG. 6B** show that seizure severity is significantly increased in vehicle treated KO mice as compared to WT as shown by a higher Racine Score. Treatment with AAV9/UsP-hSLC13A5opt in KO mice restored seizure severity to WT levels. **FIG 6C** show that the increased seizure frequency and severity in KO mice was associated with a lower percentage of mice surviving PTZ injections as compared to WT mice. Treatment with AAV9/UsP-hSLC13A5opt in KO mice protected against seizure-related deaths and restored survival to WT levels. Data presented as mean ± SEM. *p<0.05 two-way analysis of variance (ANOVA) with Sidak's multiple comparison test. n=8 WT+Vehicle, n=9 KO+Vehicle, n=6 KO+AAV9/hSLC13A5opt.

### DETAILED DESCRIPTION OF THE INVENTION

The present invention is explained in greater detail below. This description is not intended to be a detailed catalog of all the different ways in which the invention may be implemented, or all the features that may be added to the instant invention. For example, features illustrated with respect to one embodiment may be incorporated into other embodiments, and features illustrated with respect to a particular embodiment may be deleted from that embodiment. In addition, numerous variations and additions to the various embodiments suggested herein will be apparent to those skilled in the art in light of the instant disclosure which do not depart from the instant invention. Hence, the following specification is intended to illustrate some particular embodiments of the invention, and not to exhaustively specify all permutations, combinations and variations thereof.

Unless the context indicates otherwise, it is specifically intended that the various features of the invention described herein can be used in any combination. Moreover, the present invention also contemplates that in some embodiments of the invention, any feature or combination of features set forth herein can be excluded or omitted. To illustrate, if the specification states that a complex comprises components A, B and C, it is specifically intended that any of A, B or C, or a combination thereof, can be omitted and disclaimed singularly or in any combination.

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention.

Nucleotide sequences are presented herein by single strand only, in the 5' to 3' direction, from left to right, unless specifically indicated otherwise. Nucleotides and amino acids are represented herein in the manner recommended by the IUPAC-IUB Biochemical Nomenclature Commission, or (for amino acids) by either the one-letter code, or the three letter code, both in accordance with 37 C.F.R. §1.822 and established usage.

Except as otherwise indicated, standard methods known to those skilled in the art may be used for production of recombinant and synthetic polypeptides, antibodies or antigen-binding fragments thereof, manipulation of nucleic acid sequences, production of transformed cells, the construction of rAAV constructs, modified capsid proteins, packaging vectors expressing the AAV rep and/or cap sequences, and transiently and stably transfected packaging cells. Such techniques are known to those skilled in the art. *See,* e.g., SAMBROOK et al. MOLECULAR CLONING: A LABORATORY MANUAL 4th Ed. (Cold Spring Harbor, NY, 2012); F. M. AUSUBEL et al. CURRENT PROTOCOLS IN MOLECULAR BIOLOGY (Green Publishing Associates, Inc. and John Wiley & Sons, Inc., New York).

All publications, patent applications, patents, nucleotide sequences, amino acid sequences and other references mentioned herein are incorporated by reference in their entirety.

### Definitions

As used in the description of the invention and the appended claims, the singular forms "a," "an" and "the" are intended to include the plural forms as well, unless the context clearly indicates otherwise.

As used herein, "and/or" refers to and encompasses any and all possible combinations of one or more of the associated listed items, as well as the lack of combinations when interpreted in the alternative ("or").

Moreover, the present invention also contemplates that in some embodiments of the invention, any feature or combination of features set forth herein can be excluded or omitted.

Furthermore, the term "about," as used herein when referring to a measurable value such as an amount of a compound or agent of this invention, dose, time, temperature, and the like, is meant to encompass variations of ± 10%, ± 5%, ± 1%, ± 0.5%, or even ± 0.1% of the specified amount.

As used herein, the transitional phrase "consisting essentially of" is to be interpreted as encompassing the recited materials or steps and those that do not materially affect the basic and novel characteristic(s) of the claimed invention. Thus, the term "consisting essentially of" as used herein should not be interpreted as equivalent to "comprising."

The term "consists essentially of" (and grammatical variants), as applied to a polynucleotide or polypeptide sequence of this invention, means a polynucleotide or polypeptide that consists of both the recited sequence (e.g., SEQ ID NO) and a total of ten or less (e.g., 1, 2, 3, 4, 5, 6, 7, 8, 9, or 10) additional nucleotides or amino acids on the 5' and/or 3' or N-terminal and/or C-terminal ends of the recited sequence or between the two ends (e.g., between domains) such that the function of the polynucleotide or polypeptide is not materially altered. The total of ten or less additional nucleotides or amino acids includes the total number of additional nucleotides or amino acids added together. The term "materially altered," as applied to polynucleotides of the invention, refers to an increase or decrease in ability to express the encoded polypeptide of at least about 50% or more as compared to the expression level of a polynucleotide consisting of the recited sequence. The term "materially altered," as applied to polypeptides of the invention, refers to an increase or decrease in biological activity of at least about 50% or more as compared to the activity of a polypeptide consisting of the recited sequence.

The term "parvovirus" as used herein encompasses the family *Parvoviridae,* including autonomously-replicating parvoviruses and dependoviruses. The autonomous parvoviruses include members of the genera *Parvovirus, Erythrovirus, Densovirus, Iteravirus,* and *Contravirus.* Exemplary autonomous parvoviruses include, but are not limited to, minute virus of mouse, bovine parvovirus, canine parvovirus, chicken parvovirus, feline panleukopenia virus, feline parvovirus, goose parvovirus, H1 parvovirus, muscovy duck parvovirus, snake parvovirus, and B19 virus. Other autonomous parvoviruses are known to those skilled in the art. *See,* e.g., FIELDS et al. VIROLOGY, volume 2, chapter 69 (4th ed., Lippincott-Raven Publishers).

The genus *Dependovirus* contains the adeno-associated viruses (AAV), including but not limited to, AAV type 1, AAV type 2, AAV type 3 (including types 3A and 3B), AAV type 4, AAV type 5, AAV type 6, AAV type 7, AAV type 8, AAV type 9, AAV type 10, AAV type 11, AAV type 12, AAV type 13, avian AAV, bovine AAV, canine AAV, goat AAV, snake AAV, equine AAV, and ovine AAV. *See,* e.g., FIELDS et al. VIROLOGY, volume 2, chapter 69 (4th ed., Lippincott-Raven Publishers); and **Table 1.**

The term "adeno-associated virus" (AAV) in the context of the present invention includes without limitation AAV type 1, AAV type 2, AAV type 3 (including types 3A and 3B), AAV type 4, AAV type 5, AAV type 6, AAV type 7, AAV type 8, AAV type 9, AAV type 10, AAV type 11, avian AAV, bovine AAV, canine AAV, equine AAV, and ovine AAV and any other AAV now known or later discovered. *See,* e.g., BERNARD N. FIELDS et al. VIROLOGY, volume 2, chapter 69 (4th ed., Lippincott-Raven Publishers). A number of additional AAV serotypes and clades have been identified *(see,* e.g., Gao et al. 2004 J. Virol. 78:6381-6388 and **Table 1),** which are also encompassed by the term "AAV."

The parvovirus particles and genomes of the present invention can be from, but are not limited to, AAV. The genomic sequences of various serotypes of AAV and the autonomous parvoviruses, as well as the sequences of the native ITRs, Rep proteins, and capsid subunits are known in the art. Such sequences may be found in the literature or in public databases such as GenBank. *See,* e.g., GenBank Accession Numbers NC_002077, NC_001401, NC_001729, NC_001863, NC_001829, NC_001862, NC_000883, NC_001701, NC_001510, NC_006152, NC_006261, AF063497, U89790, AF043303, AF028705, AF028704, J02275, J01901, J02275, X01457, AF288061, AH009962, AY028226, AY028223, AY631966, AX753250, EU285562, NC_001358, NC_001540, AF513851, AF513852 and AY530579; the disclosures of which are incorporated by reference herein for teaching parvovirus and AAV nucleic acid and amino acid sequences. *See also,* e.g., Bantel-Schaal et al. 1999 J. Virol. 73: 939; Chiorini et al.1997) J. Virol. 71:6823; Chiorini et al. 1999 J. Virol. 73:1309; Gao et al. 2002 Proc. Nat. Acad. Sci. USA 99:11854; Moris et al. 2004 Virol. 33-:375-383; Mori et al. 2004 Virol. 330:375; Muramatsu et al. 1996 Virol. 221:208; Ruffing et al. 1994 J. Gen. Virol. 75:3385; Rutledge et al. 1998 J. Virol. 72:309; Schmidt et al. 2008 J. Virol. 82:8911; Shade et al. 1986 J. Virol. 58:921; Srivastava et al. 1983 J. Virol. 45:555; Xiao et al. 1999 J. Virol. 73:3994; international patent publications WO 00/28061, WO 99/61601, WO 98/11244; and U.S. Patent No. 6,156,303; the disclosures of which are incorporated by reference herein for teaching parvovirus and AAV nucleic acid and amino acid sequences. *See also* **Table 1**. An early description of the AAV1, AAV2 and AAV3 ITR sequences is provided by Xiao, X. 1996 "Characterization of Adeno-associated virus (AAV) DNA replication and integration," Ph.D. Dissertation, University of Pittsburgh, Pittsburgh, PA (incorporated herein it its entirety).

A "chimeric" AAV nucleic acid capsid coding sequence or AAV capsid protein is one that combines portions of two or more capsid sequences. A "chimeric" AAV virion or particle comprises a chimeric AAV capsid protein.

The term "tropism" as used herein refers to preferential entry of the virus into certain cell or tissue type(s) and/or preferential interaction with the cell surface that facilitates entry into certain cell or tissue types, optionally and preferably followed by expression (e.g., transcription and, optionally, translation) of sequences carried by the viral genome in the cell, e.g., for a recombinant virus, expression of the heterologous nucleotide sequence(s). Those skilled in the art will appreciate that transcription of a heterologous nucleic acid sequence from the viral genome may not be initiated in the absence of trans-acting factors, e.g., for an inducible promoter or otherwise regulated nucleic acid sequence. In the case of a rAAV genome, gene expression from the viral genome may be from a stably integrated provirus and/or from a non-integrated episome, as well as any other form which the virus nucleic acid may take within the cell.

The term "tropism profile" refers to the pattern of transduction of one or more target cells, tissues and/or organs. Representative examples of chimeric AAV capsids have a tropism profile characterized by efficient transduction of cells of the central nervous system (CNS) with only low transduction of peripheral organs (*see* e.g., US Patent No. 9,636,370 McCown et al. and US patent publication 2017/0360960 Gray et al.).

The term "disorder associated with aberrant expression of a SLC13A5 gene" as used herein refers to a disease, disorder, syndrome, or condition that is caused by or a symptom of decreased or altered expression of the SLC13A5 gene in a subject relative to the expression level in a normal subject or in a population.

The term "disorder associated with aberrant activity of a SLC13A5 gene product" as used herein refers to a disease, disorder, syndrome, or condition that is caused by or a symptom of decreased or altered activity of the SLC13A5 gene product in a subject relative to the activity in a normal subject or in a population. In some embodiments, a disorder associated with aberrant activity of a SLC13A5 gene product may be a citrate transporter disorder, e.g., SLC13A5 deficiency.

As used herein, "transduction" of a cell by a virus vector (e.g., an AAV vector) means entry of the vector into the cell and transfer of genetic material into the cell by the incorporation of nucleic acid into the virus vector and subsequent transfer into the cell via the virus vector.

Unless indicated otherwise, "efficient transduction" or "efficient tropism," or similar terms, can be determined by reference to a suitable positive or negative control (e.g., at least about 50%, 60%, 70%, 80%, 85%, 90%, 95% or more of the transduction or tropism, respectively, of a positive control or at least about 110%, 120%, 150%, 200%, 300%, 500%, 1000% or more of the transduction or tropism, respectively, of a negative control).

**Table 1**

| **AAV Serotypes/Isolates** | **GenBank Accession Number** | | **AAV Serotypes/Isolates** | **GenBank Accession Number** | | **AAV Serotypes/Isolates** | **GenBank Accession Number** |
|---|---|---|---|---|---|---|---|
| **Clonal Isolates** | | | Hu S17 | AY695376 | | Cy3 | AY243019 |
| Avian AAV ATCC VR-865 | AY186198, AY629583, NC_004828 | | Hu T88 | AY695375 | | Cy5 | AY243017 |
| Avian AAV strain DA-1 | NC_006263, AY629583 | | Hu T71 | AY695374 | | Rh13 | AY243013 |
| Bovine AAV | NC_005889, AY388617 | | Hu T70 | AY695373 | | | |
| **AAV4** | NC 001829 | | Hu T40 | AY695372 | | **Clade E** | |
| **AAV5** | AY18065, AF085716 | | Hu T32 | AY695371 | | Rh38 | AY530558 |
| Rh34 | AY243001 | | Hu T17 | AY695370 | | Hu66 | AY530626 |
| Rh33 | AY243002 | | Hu LG15 | AY695377 | | Hu42 | AY530605 |
| Rh32 | AY243003 | | | | | Hu67 | AY530627 |
| **AAV10** | AY631965 | | **Clade C** | | | Hu40 | AY530603 |
| **AAV11** | AY631966 | | **AAV 3** | NC 001729 | | Hu41 | AY530604 |
| **AAV12** | DQ813647 | | **AAV 3B** | NC 001863 | | Hu37 | AY530600 |
| **AAV13** | EU285562 | | Hu9 | AY530629 | | Rh40 | AY530559 |
| | | | Hu10 | AY530576 | | Rh2 | AY243007 |
| **Clade A** | | | Hu11 | AY530577 | | Bb1 | AY243023 |
| **AAV1** | NC_002077, AF063497 | | Hu53 | AY530615 | | Bb2 | AY243022 |
| **AAV6** | NC 001862 | | Hu55 | AY530617 | | **Rh10** | AY243015 |
| Hu.48 | AY530611 | | Hu54 | AY530616 | | Hu17 | AY530582 |
| Hu 43 | AY530606 | | Hu7 | AY530628 | | Hu6 | AY530621 |
| Hu 44 | AY530607 | | Hu18 | AY530583 | | Rh25 | AY530557 |
| Hu 46 | AY530609 | | Hu15 | AY530580 | | Pi2 | AY530554 |
| | | | Hu16 | AY530581 | | Pi1 | AY530553 |
| **Clade B** | | | Hu25 | AY530591 | | Pi3 | AY530555 |
| Hu19 | AY530584 | | Hu60 | AY530622 | | Rh57 | AY530569 |
| Hu20 | AY530586 | | Ch5 | AY243021 | | Rh50 | AY530563 |
| Hu23 | AY530589 | | Hu3 | AY530595 | | Rh49 | AY530562 |
| Hu22 | AY530588 | | Hu1 | AY530575 | | Hu39 | AY530601 |
| Hu24 | AY530590 | | Hu4 | AY530602 | | Rh58 | AY530570 |
| Hu21 | AY530587 | | Hu2 | AY530585 | | Rh61 | AY530572 |
| Hu27 | AY530592 | | Hu61 | AY530623 | | Rh52 | AY530565 |
| Hu28 | AY530593 | | | | | Rh53 | AY530566 |
| Hu29 | AY530594 | | **Clade D** | | | Rh51 | AY530564 |
| Hu63 | AY530624 | | Rh62 | AY530573 | | Rh64 | AY530574 |
| Hu64 | AY530625 | | Rh48 | AY530561 | | Rh43 | AY530560 |
| Hu13 | AY530578 | | Rh54 | AY530567 | | **AAV8** | AF513852 |
| Hu56 | AY530618 | | Rh55 | AY530568 | | Rh8 | AY242997 |
| Hu57 | AY530619 | | Cy2 | AY243020 | | Rh1 | AY530556 |
| Hu49 | AY530612 | | **AAV7** | AF513851 | | | |
| Hu58 | AY530620 | | Rh35 | AY243000 | | **Clade F** | |
| Hu34 | AY530598 | | Rh37 | AY242998 | | **AAV9** (Hu14) | AY530579 |
| Hu35 | AY530599 | | Rh36 | AY242999 | | Hu31 | AY530596 |
| **AAV2** | NC 001401 | | Cy6 | AY243016 | | Hu32 | AY530597 |
| Hu45 | AY530608 | | Cy4 | AY243018 | | | |
| Hu47 | AY530610 | | | | | | |
| Hu51 | AY530613 | | | | | | |
| Hu52 | AY530614 | | | | | | |
| Hu T41 | AY695378 | | | | | | |

Similarly, it can be determined if a virus "does not efficiently transduce" or "does not have efficient tropism" for a target tissue, or similar terms, by reference to a suitable control. In particular embodiments, the virus vector does not efficiently transduce (i.e., does not have efficient tropism for) tissues outside the CNS, e.g., liver, kidney, gonads and/or germ cells. In particular embodiments, undesirable transduction of tissue(s) (e.g., liver) is 20% or less, 10% or less, 5% or less, 1% or less, 0.1% or less of the level of transduction of the desired target tissue(s) (e.g., CNS cells).

The terms "5' portion" and "3' portion" are relative terms to define a spatial relationship between two or more elements. Thus, for example, a "3' portion" of a polynucleotide indicates a segment of the polynucleotide that is downstream of another segment. The term "3' portion" is not intended to indicate that the segment is necessarily at the 3' end of the polynucleotide, or even that it is necessarily in the 3' half of the polynucleotide, although it may be. Likewise, a "5' portion" of a polynucleotide indicates a segment of the polynucleotide that is upstream of another segment. The term "5' portion" is not intended to indicate that the segment is necessarily at the 5' end of the polynucleotide, or even that it is necessarily in the 5' half of the polynucleotide, although it may be.

As used herein, the term "polypeptide" encompasses both peptides and proteins, unless indicated otherwise.

A "polynucleotide," "nucleic acid," or "nucleotide sequence" may be of RNA, DNA or DNA-RNA hybrid sequences (including both naturally occurring and non-naturally occurring nucleotides), but is preferably either a single or double stranded DNA sequence.

The term "regulatory element" refers to a genetic element which controls some aspect of the expression of nucleic acid sequences. For example, a promoter is a regulatory element which facilitates the initiation of transcription of an operably linked coding region. Other regulatory elements are splicing signals, polyadenylation signals, termination signals, etc. The region in a nucleic acid sequence or polynucleotide in which one or more regulatory elements are found may be referred to as a "regulatory region."

As used herein with respect to nucleic acids, the term "operably linked" refers to a functional linkage between two or more nucleic acids. For example, a promoter sequence may be described as being "operably linked" to a heterologous nucleic acid sequence because the promoter sequences initiates and/or mediates transcription of the heterologous nucleic acid sequence. In some embodiments, the operably linked nucleic acid sequences are contiguous and/or are in the same reading frame.

The term "open reading frame (ORF)," as used herein, refers to the portion of a polynucleotide, e.g., a gene, that encodes a polypeptide. The term "coding region" may be used interchangeably with open reading frame.

The term "codon-optimized," as used herein, refers to a gene coding sequence that has been optimized to increase expression by substituting one or more codons normally present in a coding sequence (for example, in a wild-type sequence, including, e.g., a coding sequence for SLC13A5) with a codon for the same (synonymous) amino acid. In this manner, the protein encoded by the gene is identical, but the underlying nucleobase sequence of the gene or corresponding mRNA is different. In some embodiments, the optimization substitutes one or more rare codons (that is, codons for tRNA that occur relatively infrequently in cells from a particular species) with synonymous codons that occur more frequently to improve the efficiency of translation. For example, in human codon-optimization one or more codons in a coding sequence are replaced by codons that occur more frequently in human cells for the same amino acid. Codon optimization can also increase gene expression through other mechanisms that can improve efficiency of transcription and/or translation. Strategies include, without limitation, increasing total GC content (that is, the percent of guanines and cytosines in the entire coding sequence), decreasing CpG content (that is, the number of CG or GC dinucleotides in the coding sequence), removing cryptic splice donor or acceptor sites, and/or adding or removing ribosomal entry sites, such as Kozak sequences. Desirably, a codon-optimized gene exhibits improved protein expression, for example, the protein encoded thereby is expressed at a detectably greater level in a cell compared with the level of expression of the protein provided by the wild-type gene in an otherwise similar cell.

The term "sequence identity," as used herein, has the standard meaning in the art. As is known in the art, a number of different programs can be used to identify whether a polynucleotide or polypeptide has sequence identity or similarity to a known sequence. Sequence identity or similarity may be determined using standard techniques known in the art, including, but not limited to, the local sequence identity algorithm of Smith & Waterman, Adv. Appl. Math. 2:482 (1981), by the sequence identity alignment algorithm of Needleman & Wunsch, J. Mol. Biol. 48:443 (1970), by the search for similarity method of Pearson & Lipman, Proc. Natl. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Drive, Madison, WI), the Best Fit sequence program described by Devereux et al. 1984 Nucl. Acid Res. 12:387, preferably using the default settings, or by inspection.

An example of a useful algorithm is PILEUP. PILEUP creates a multiple sequence alignment from a group of related sequences using progressive, pairwise alignments. It can also plot a tree showing the clustering relationships used to create the alignment. PILEUP uses a simplification of the progressive alignment method of Feng & Doolittle, J. Mol. Evol. 35:351 (1987); the method is similar to that described by Higgins & Sharp, CABIOS 5:151 (1989).

Another example of a useful algorithm is the BLAST algorithm, described in Altschul et al. 1990 J. Mol. Biol. 215:403 and Karlin et al. 1993 Proc. Natl. Acad. Sci. USA 90:5873. A particularly useful BLAST program is the WU-BLAST-2 program which was obtained from Altschul et al. 1996 Meth. Enzymol., 266:460; blast.wustl/edu/blast/README.html. WU-BLAST-2 uses several search parameters, which are preferably set to the default values. The parameters are dynamic values and are established by the program itself depending upon the composition of the particular sequence and composition of the particular database against which the sequence of interest is being searched; however, the values may be adjusted to increase sensitivity.

An additional useful algorithm is gapped BLAST as reported by Altschul et al. 1997 Nucleic Acids Res. 25:3389*.*

A percentage amino acid sequence identity value is determined by the number of matching identical residues divided by the total number of residues of the "longer" sequence in the aligned region. The "longer" sequence is the one having the most actual residues in the aligned region (gaps introduced by WU-Blast-2 to maximize the alignment score are ignored).

In a similar manner, percent nucleic acid sequence identity is defined as the percentage of nucleotide residues in the candidate sequence that are identical with the nucleotides in the polynucleotide specifically disclosed herein.

The alignment may include the introduction of gaps in the sequences to be aligned. In addition, for sequences which contain either more or fewer nucleotides than the polynucleotides specifically disclosed herein, it is understood that in one embodiment, the percentage of sequence identity will be determined based on the number of identical nucleotides in relation to the total number of nucleotides. Thus, for example, sequence identity of sequences shorter than a sequence specifically disclosed herein, will be determined using the number of nucleotides in the shorter sequence, in one embodiment. In percent identity calculations relative weight is not assigned to various manifestations of sequence variation, such as insertions, deletions, substitutions, etc.

In one embodiment, only identities are scored positively (+1) and all forms of sequence variation including gaps are assigned a value of "0," which obviates the need for a weighted scale or parameters as described below for sequence similarity calculations. Percent sequence identity can be calculated, for example, by dividing the number of matching identical residues by the total number of residues of the "shorter" sequence in the aligned region and multiplying by 100. The "longer" sequence is the one having the most actual residues in the aligned region.

As used herein, an "isolated" nucleic acid or nucleotide sequence (e.g., an "isolated DNA" or an "isolated RNA") means a nucleic acid or nucleotide sequence separated or substantially free from at least some of the other components of the naturally occurring organism or virus, for example, the cell or viral structural components or other polypeptides or nucleic acids commonly found associated with the nucleic acid or nucleotide sequence.

Likewise, an "isolated" polypeptide means a polypeptide that is separated or substantially free from at least some of the other components of the naturally occurring organism or virus, for example, the cell or viral structural components or other polypeptides or nucleic acids commonly found associated with the polypeptide.

As used herein, the term "modified," as applied to a polynucleotide or polypeptide sequence, refers to a sequence that differs from a wild-type sequence due to one or more deletions, additions, substitutions, or any combination thereof.

As used herein, by "isolate" (or grammatical equivalents) a virus vector, it is meant that the virus vector is at least partially separated from at least some of the other components in the starting material.

By the term "treat," "treating," or "treatment of" (or grammatically equivalent terms) is meant to reduce or to at least partially improve or ameliorate the severity of the subject's condition and/or to alleviate, mitigate or decrease in at least one clinical symptom and/or to delay the progression of the condition.

As used herein, the term "prevent," "prevents," or "prevention" (and grammatical equivalents thereof) means to delay or inhibit the onset of a disease. The terms are not meant to require complete abolition of disease, and encompass any type of prophylactic treatment to reduce the incidence of the condition or delays the onset of the condition.

A "treatment effective" or "therapeutically effective" amount as used herein is an amount that is sufficient to provide some improvement or benefit to the subject. Alternatively stated, a "treatment effective" amount is an amount that will provide some alleviation, mitigation, decrease or stabilization in at least one clinical symptom in the subject. Those skilled in the art will appreciate that the therapeutic effects need not be complete or curative, as long as some benefit is provided to the subject.

A "prevention effective" amount as used herein is an amount that is sufficient to prevent and/or delay the onset of a disease, disorder and/or clinical symptoms in a subject and/or to reduce and/or delay the severity of the onset of a disease, disorder and/or clinical symptoms in a subject relative to what would occur in the absence of the methods of the invention. Those skilled in the art will appreciate that the level of prevention need not be complete, as long as some benefit is provided to the subject.

A "heterologous nucleotide sequence" or "heterologous nucleic acid," with respect to a virus, is a sequence or nucleic acid, respectively, that is not naturally occurring in the virus. Generally, the heterologous nucleic acid or nucleotide sequence comprises an open reading frame that encodes a polypeptide and/or a nontranslated RNA.

A "vector" refers to a compound used as a vehicle to carry foreign genetic material into another cell, where it can be replicated and/or expressed. A cloning vector containing foreign nucleic acid is termed a recombinant vector. Examples of nucleic acid vectors are plasmids, viral vectors, cosmids, expression cassettes, and artificial chromosomes. Recombinant vectors typically contain an origin of replication, a multicloning site, and a selectable marker. The nucleic acid sequence typically consists of an insert (recombinant nucleic acid or transgene) and a larger sequence that serves as the "backbone" of the vector. The purpose of a vector which transfers genetic information to another cell is typically to isolate, multiply, or express the insert in the target cell. Expression vectors (expression constructs or expression cassettes) are for the expression of the exogenous gene in the target cell, and generally have a promoter sequence that drives expression of the exogenous gene/ORF. Insertion of a vector into the target cell is referred to transformation or transfection for bacterial and eukaryotic cells, although insertion of a viral vector is often called transduction. The term "vector" may also be used in general to describe items to that serve to carry foreign genetic material into another cell, such as, but not limited to, a transformed cell or a nanoparticle.

As used herein, the term "vector," "virus vector," "delivery vector" (and similar terms) in a specific embodiment generally refers to a virus particle that functions as a nucleic acid delivery vehicle, and which comprises the viral nucleic acid (i.e., the vector genome) packaged within the virion. Virus vectors according to the present invention comprise a chimeric AAV capsid according to the invention and can package an AAV or rAAV genome or any other nucleic acid including viral nucleic acids. Alternatively, in some contexts, the term "vector," "virus vector," "delivery vector" (and similar terms) may be used to refer to the vector genome (e.g., vDNA) in the absence of the virion and/or to a viral capsid that acts as a transporter to deliver molecules tethered to the capsid or packaged within the capsid.

The virus vectors of the invention can further be duplexed parvovirus particles as described in international patent publication WO 01/92551 (the disclosure of which is incorporated herein by reference in its entirety). Thus, in some embodiments, double stranded (duplex) genomes can be packaged.

A "recombinant AAV vector genome" or "rAAV genome" is an AAV genome (i.e., vDNA) that comprises at least one inverted terminal repeat (e.g., one, two or three inverted terminal repeats) and one or more heterologous nucleotide sequences. rAAV vectors generally retain the 145 base terminal repeat(s) (TR(s)) in *cis* to generate virus; however, modified AAV TRs and non-AAV TRs including partially or completely synthetic sequences can also serve this purpose. All other viral sequences are dispensable and may be supplied in *trans* (Muzyczka, (1992) Curr. Topics Microbiol. Immunol. 158:97). The rAAV vector optionally comprises two TRs (e.g., AAV TRs), which generally will be at the 5' and 3' ends of the heterologous nucleotide sequence(s), but need not be contiguous thereto. The TRs can be the same or different from each other. The vector genome can also contain a single ITR at its 3' or 5' end.

The term "terminal repeat" or "TR" includes any viral terminal repeat or synthetic sequence that forms a hairpin structure and functions as an inverted terminal repeat (ITR) (i.e., mediates the desired functions such as replication, virus packaging, integration and/or provirus rescue, and the like). The TR can be an AAV TR or a non-AAV TR. For example, a non-AAV TR sequence such as those of other parvoviruses (e.g., canine parvovirus (CPV), mouse parvovirus (MVM), human parvovirus B-19) or the SV40 hairpin that serves as the origin of SV40 replication can be used as a TR, which can further be modified by truncation, substitution, deletion, insertion and/or addition. Further, the TR can be partially or completely synthetic, such as the "double-D sequence" as described in United States Patent No. 5,478,745 to Samulski et al.

Parvovirus genomes have palindromic sequences at both their 5' and 3' ends. The palindromic nature of the sequences leads to the formation of a hairpin structure that is stabilized by the formation of hydrogen bonds between the complementary base pairs. This hairpin structure is believed to adopt a "Y" or a "T" shape. *See,* e.g., FIELDS et al. VIROLOGY, volume 2, chapters 69 & 70 (4th ed., Lippincott-Raven Publishers).

An "AAV inverted terminal repeat" or "AAV ITR" may be from any AAV, including but not limited to serotypes 1, 2, 3, 4, 5, 6, 7, 8, 9, 10 or 11 or any other AAV now known or later discovered *(see,* e.g., **Table** 1). An AAV ITR need not have the native ITR sequence (e.g., a native AAV ITR sequence may be altered by insertion, deletion, truncation and/or missense mutations), as long as the ITR mediates the desired functions, e.g., replication, virus packaging, integration, and/or provirus rescue, and the like.

The terms "rAAV particle" and "rAAV virion" are used interchangeably here. A "rAAV particle" or "rAAV virion" comprises a rAAV vector genome packaged within an AAV capsid.

The virus vectors of the invention can further be "targeted" virus vectors (e.g., having a directed tropism) and/or a "hybrid" parvovirus (i.e., in which the viral ITRs and viral capsid are from different parvoviruses) as described in international patent publication WO 00/28004 and Chao et al. 2000 Mol. Therapy 2:619*.*

Further, the viral capsid or genomic elements can contain other modifications, including insertions, deletions and/or substitutions.

As used herein, the term "amino acid" encompasses any naturally occurring amino acids, modified forms thereof, and synthetic amino acids, including non-naturally occurring amino acids. Naturally occurring, levorotatory (L-) amino acids are shown in **Table 2.**

**Table 2**

| **Amino Acid Residue** | **Abbreviation** | |
|---|---|---|
| | **Three-Letter Code** | **One-Letter Code** |
| Alanine | Ala | A |
| Arginine | Arg | R |
| Asparagine | Asn | N |
| Aspartic acid (Aspartate) | Asp | D |
| Cysteine | Cys | C |
| Glutamine | Gln | Q |
| Glutamic acid (Glutamate) | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

Alternatively, the amino acid can be a modified amino acid residue (nonlimiting examples are shown in **Table 3)** or can be an amino acid that is modified by post-translation modification (e.g., acetylation, amidation, formylation, hydroxylation, methylation, phosphorylation or sulfatation).

**Table 3: Amino Acid Residue Derivatives**

| **Modified Amino Acid Residue** | **Abbreviation** | |
|---|---|---|
| 2-Aminoadipic acid | Aad | |
| 3-Aminoadipic acid | bAad | |
| beta-Alanine, beta-Aminoproprionic acid | bAla | |
| 2-Aminobutyric acid | Abu | |
| 4-Aminobutyric acid, Piperidinic acid | 4Abu | |
| 6-Aminocaproic acid | Acp | |
| 2-Aminoheptanoic acid | Ahe | |
| 2-Aminoisobutyric acid | Aib | |
| 3-Aminoisobutyric acid | bAib | |
| 2-Aminopimelic acid | Apm | |
| t-butylalanine | t-BuA | |
| Citrulline | Cit | |
| Cyclohexylalanine | Cha | |
| 2,4-Diaminobutyric acid | Dbu | |
| Desmosine | Des | |
| 2,2'-Diaminopimelic acid | Dpm | |
| 2,3-Diaminoproprionic acid | Dpr | |
| N-Ethylglycine | EtGly | |
| N-Ethylasparagine | EtAsn | |
| Homoarginine | hArg | |
| Homocysteine | hCys | |
| Homoserine | hSer | |
| Hydroxylysine | Hyl | |
| Allo-Hydroxylysine | aHyl | |
| 3-Hydroxyproline | 3Hyp | |
| 4-Hydroxyproline | 4Hyp | |
| Isodesmosine | Ide | |
| allo-Isoleucine | aIle | |
| Methionine sulfoxide | MSO | |
| N-Methylglycine, sarcosine | MeGly | |
| N-Methylisoleucine | MeIle | |
| 6-N-Methyllysine | MeLys | |
| N-Methylvaline | MeVal | |
| 2-Naphthylalanine | 2-Nal | |
| Norvaline | Nva | |
| Norleucine | Nle | |
| Ornithine | | Orn |
| 4-Chlorophenylalanine | | Phe(4-Cl) |
| 2-Fluorophenylalanine | | Phe(2-F) |
| 3-Fluorophenylalanine | | Phe(3-F) |
| 4-Fluorophenylalanine | | Phe(4-F) |
| Phenylglycine | Phg | |
| Beta-2-thienylalanine | Thi | |

Further, the non-naturally occurring amino acid can be an "unnatural" amino acid as described by Wang et al. 2006 Annu. Rev. Biophys. Biomol. Struct. 35:225-49. These unnatural amino acids can advantageously be used to chemically link molecules of interest to the AAV capsid protein.

The term "template" or "substrate" is used herein to refer to a polynucleotide sequence that may be replicated to produce the parvovirus viral DNA. For the purpose of vector production, the template will typically be embedded within a larger nucleotide sequence or construct, including but not limited to a plasmid, naked DNA vector, bacterial artificial chromosome (BAC), yeast artificial chromosome (YAC) or a viral vector (e.g., adenovirus, herpesvirus, Epstein-Barr Virus, AAV, baculoviral, retroviral vectors, and the like). Alternatively, the template may be stably incorporated into the chromosome of a packaging cell.

As used herein, parvovirus or AAV "Rep coding sequences" indicate the nucleic acid sequences that encode the parvoviral or AAV non-structural proteins that mediate viral replication and the production of new virus particles. The parvovirus and AAV replication genes and proteins have been described in, e.g., FIELDS et al. VIROLOGY, volume 2, chapters 69 & 70 (4th ed., Lippincott-Raven Publishers).

The "Rep coding sequences" need not encode all of the parvoviral or AAV Rep proteins. For example, with respect to AAV, the Rep coding sequences do not need to encode all four AAV Rep proteins (Rep78, Rep 68, Rep52 and Rep40), in fact, it is believed that AAV5 only expresses the spliced Rep68 and Rep40 proteins. In representative embodiments, the Rep coding sequences encode at least those replication proteins that are necessary for viral genome replication and packaging into new virions. The Rep coding sequences will generally encode at least one large Rep protein (i.e., Rep78/68) and one small Rep protein (i.e., Rep52/40). In particular embodiments, the Rep coding sequences encode the AAV Rep78 protein and the AAV Rep52 and/or Rep40 proteins. In other embodiments, the Rep coding sequences encode the Rep68 and the Rep52 and/or Rep40 proteins. In a still further embodiment, the Rep coding sequences encode the Rep68 and Rep52 proteins, Rep68 and Rep40 proteins, Rep78 and Rep52 proteins, or Rep78 and Rep40 proteins.

As used herein, the term "large Rep protein" refers to Rep68 and/or Rep78. Large Rep proteins of the claimed invention may be either wild-type or synthetic. A wild-type large Rep protein may be from any parvovirus or AAV, including but not limited to serotypes 1, 2, 3a, 3b, 4, 5, 6, 7, 8, 9, 10, 11, or 13, or any other AAV now known or later discovered *(see,* e.g., **Table 1).** A synthetic large Rep protein may be altered by insertion, deletion, truncation and/or missense mutations.

Those skilled in the art will further appreciate that it is not necessary that the replication proteins be encoded by the same polynucleotide. For example, for MVM, the NS-1 and NS-2 proteins (which are splice variants) may be expressed independently of one another. Likewise, for AAV, the p19 promoter may be inactivated and the large Rep protein(s) expressed from one polynucleotide and the small Rep protein(s) expressed from a different polynucleotide. Typically, however, it will be more convenient to express the replication proteins from a single construct. In some systems, the viral promoters (e.g., AAV p19 promoter) may not be recognized by the cell, and it is therefore necessary to express the large and small Rep proteins from separate expression cassettes. In other instances, it may be desirable to express the large Rep and small Rep proteins separately, i.e., under the control of separate transcriptional and/or translational control elements. For example, it may be desirable to control expression of the large Rep proteins, so as to decrease the ratio of large to small Rep proteins. In the case of insect cells, it may be advantageous to down-regulate expression of the large Rep proteins (e.g., Rep78/68) to avoid toxicity to the cells *(see,* e.g., Urabe et al. 2002 Human Gene Therapy 13:1935).

As used herein, the parvovirus or AAV "cap coding sequences" encode the structural proteins that form a functional parvovirus or AAV capsid (i.e., can package DNA and infect target cells). Typically, the cap coding sequences will encode all of the parvovirus or AAV capsid subunits, but less than all of the capsid subunits may be encoded as long as a functional capsid is produced. Typically, but not necessarily, the cap coding sequences will be present on a single nucleic acid molecule.

The capsid structure of autonomous parvoviruses and AAV are described in more detail in BERNARD N. FIELDS et al. VIROLOGY, volume 2, chapters 69 & 70 (4th ed., Lippincott-Raven Publishers).

By "substantially retain" a property, it is meant that at least about 75%, 85%, 90%, 95%, 97%, 98%, 99% or 100% of the property (e.g., activity or other measurable characteristic) is retained.

### SLC13A5 Expression Cassettes and Vectors

The present invention relates to the design of a SLC13A5 expression cassette to provide therapeutic levels of expression of Na⁺/citrate cotransporter, the protein encoded by the SLC13A5 gene, and the use of the expression cassette to achieve therapeutic levels of SLC13A5 and/or Na⁺/citrate cotransporter in a subject.

Thus, one aspect of the invention relates to a polynucleotide comprising a mammalian SLC13A5 open reading frame (ORF), wherein the SLC13A5 open reading frame has been codon-optimized for expression in mammalian cells. The term "mammal" as used herein includes, but is not limited to, humans, primates, non-human primates (e.g., monkeys and baboons), cattle, sheep, goats, pigs, horses, cats, dogs, rabbits, rodents (e.g., rats, mice, hamsters, and the like), etc. The open reading frame is the portion of the SLC13A5 gene that encodes Na⁺/citrate cotransporter. In some embodiments, the mammalian SLC13A5 open reading frame may be a human SLC13A5 open reading frame. As used herein, a mammalian SLC13A5 ORF refers to a nucleotide sequence that encodes mammalian Na⁺/citrate cotransporter, e.g., a human SLC13A5 ORF refers to a nucleotide sequence that encodes a human Na⁺/citrate cotransporter. Codon optimization is a technique well known in the art and optimal codons for expression in different species are known. The use of a codon-optimized SLC13A5 sequence allows one to distinguish expression of the transduced sequence from expression of the endogenous SLC13A5 sequence in a subject.

In some embodiments, the codon-optimized SLC13A5 open reading frame encodes an Na⁺/citrate cotransporter protein that is modified from the wild-type sequence, e.g., comprises, consists essentially of, or consists of an amino acid sequence in which 1, 2, 3, 4, or 5 residues have been substituted, added, and/or deleted compared to the wild-type amino acid sequence.

SEQ ID NO:1. Human SLC13A5 (CCDS 11079.1; NCBI Accession No. NM_177550.5)
ATGGCCTCGGCGCTGAGCTATGTCTCCAAGTTCAAGTCCTTCGTGATCTTGTTCGT
CACCCCGCTCCTGCTGCTGCCACTCGTCATTCTGATGCCCGCCAAGTTTGTCAGGT
GTGCCTACGTCATCATCCTCATGGCCATTTACTGGTGCACAGAAGTCATCCCTCT
GGCTGTCACCTCTCTCATGCCTGTCTTGCTTTTCCCACTCTTCCAGATTCTGGACT
CCAGGCAGGTGTGTGTCCAGTACATGAAGGACACCAACATGCTGTTCCTGGGCG
GCCTCATCGTGGCCGTGGCTGTGGAGCGCTGGAACCTGCACAAGAGGATCGCCC
TGCGCACGCTCCTCTGGGTGGGGGCCAAGCCTGCACGGCTGATGCTGGGCTTCAT
GGGCGTCACAGCCCTCCTGTCCATGTGGATCAGTAACACGGCAACCACGGCCAT
GATGGTGCCCATCGTGGAGGCCATATTGCAGCAGATGGAAGCCACAAGCGCAGC
CACCGAGGCCGGCCTGGAGCTGGTGGACAAGGGCAAGGCCAAGGAGCTGCCAG
GGAGTCAAGTGATTTTTGAAGGCCCCACTCTGGGGCAGCAGGAAGACCAAGAGC
GGAAGAGGTTGTGTAAGGCCATGACCCTGTGCATCTGCTACGCGGCCAGCATCG
GGGGCACCGCCACCCTGACCGGGACGGGACCCAACGTGGTGCTCCTGGGCCAGA
TGAACGAGTTGTTTCCTGACAGCAAGGACCTCGTGAACTTTGCTTCCTGGTTTGC

ATTTGCCTTTCCCAACATGCTGGTGATGCTGCTGTTCGCCTGGCTGTGGCTCCAGT
TTGTTTACATGAGATTCAATTTTAAAAAGTCCTGGGGCTGCGGGCTAGAGAGCAA
GAAAAACGAGAAGGCTGCCCTCAAGGTGCTGCAGGAGGAGTACCGGAAGCTGG
GGCCCTTGTCCTTCGCGGAGATCAACGTGCTGATCTGCTTCTTCCTGCTGGTCATC
CTGTGGTTCTCCCGAGACCCCGGCTTCATGCCCGGCTGGCTGACTGTTGCCTGGG
TGGAGGGTGAGACAAAGTATGTCTCCGATGCCACTGTGGCCATCTTTGTGGCCAC
CCTGCTATTCATTGTGCCTTCACAGAAGCCCAAGTTTAACTTCCGCAGCCAGACT
GAGGAAGAAAGGAAAACTCCATTTTATCCCCCTCCCCTGCTGGATTGGAAGGTA
ACCCAGGAGAAAGTGCCCTGGGGCATCGTGCTGCTACTAGGGGGCGGATTTGCT
CTGGCTAAAGGATCCGAGGCCTCGGGGCTGTCCGTGTGGATGGGGAAGCAGATG
GAGCCCTTGCACGCAGTGCCCCCGGCAGCCATCACCTTGATCTTGTCCTTGCTCG
TTGCCGTGTTCACTGAGTGCACAAGCAACGTGGCCACCACCTTGTTCCTGCC
CATCTTTGCCTCCATGTCTCGCTCCATCGGCCTCAATCCGCTGTACATCATGCTGC
CCTGTACCCTGAGTGCCTCCTTTGCCTTCATGTTGCCTGTGGCCACCCCTCCAAAT
GCCATCGTGTTCACCTATGGGCACCTCAAGGTTGCTGACATGGTGAAAACAGGA
GTCATAATGAACATAATTGGAGTCTTCTGTGTGTTTTTGGCTGTCAACACCTGGG
GACGGGCCATATTTGACTTGGATCATTTCCCTGACTGGGCTAATGTGACACATAT
TGAGACT

In some embodiments, the codon-optimized SLC13A5 open reading frame comprises, consists essentially of, or consists of the nucleotide sequence of SEQ ID NO:2 or a sequence at least about 70% identical thereto, e.g., at least about 70, 75, 80, 85, 90, 91, 92, 93, 94, 95, 96, 97, 98, or 99% identical thereto.

SEQ ID NO:2. Human codon-optimized SLC13A5 open reading frame
ATGGCCTCGGCGTTGAGCTACGTGTCCAAGTTCAAGTCCTTTGTGATCCTGTTTGT
GACCCCTCTCTTGCTCCTGCCACTCGTGATTCTGATGCCGGCCAAGTTTGTGCGCT
GCGCCTACGTGATCATCCTGATGGCAATCTACTGGTGTACCGAAGTCATCCCCCT
CGCCGTGACTTCTCTTATGCCGGTGCTGCTGTTCCCGCTGTTCCAAATCCTCGACA
GCCGCCAGGTCTGCGTGCAGTACATGAAGGACACCAACATGCTGTTCCTGGGCG
GCCTGATTGTGGCCGTGGCAGTGGAGCGGTGGAACCTCCACAAGCGCATCGCCC
TGAGGACTCTGCTCTGGGTCGGCGCTAAGCCAGCGCGGCTGATGCTGGGGTTCAT
GGGAGTGACCGCGCTGCTCTCCATGTGGATCAGCAACACCGCCACTACCGCCAT
GATGGTCCCGATCGTGGAAGCCATTCTGCAACAGATGGAAGCTACTTCCGCCGCC
ACTGAAGCGGGACTGGAACTGGTGGACAAGGGAAAGGCCAAGGAACTGCCCGG
TTCACAAGTCATCTTCGAGGGACCAACTCTGGGCCAGCAGGAGGACCAAGAGCG

CAAGCGGCTGTGCAAGGCCATGACCTTGTGTATCTGCTACGCCGCTTCGATCGGA
GGCACTGCCACCCTGACCGGGACTGGACCCAACGTCGTGCTGCTGGGACAGATG
AACGAGCTGTTCCCGGATTCCAAGGACCTTGTGAACTTCGCGTCGTGGTTCGCCT
TCGCATTCCCTAACATGCTCGTGATGCTGCTCTTTGCATGGCTGTGGCTGCAGTTC
GTGTATATGCGGTTTAACTTCAAGAAGTCCTGGGGATGCGGTCTGGAATCCAAGA
AGAACGAGAAGGCCGCGCTTAAGGTCCTCCAGGAAGAATACCGGAAATTGGGGC
CGCTGAGCTTCGCCGAGATCAACGTGCTCATCTGTTTCTTCCTGCTGGTCATCCTG
TGGTTCTCAAGAGATCCTGGTTTCATGCCGGGGTGGCTCACTGTGGCCTGGGTCG
AAGGAGAAACCAAATACGTGTCGGATGCGACTGTGGCAATCTTTGTCGCCACGC
TCCTGTTCATCGTGCCTTCCCAAAAGCCTAAGTTCAACTTCCGCTCCCAAACCGA
AGAGGAGCGCAAAACACCGTTTTACCCTCCACCTCTGTTGGACTGGAAAGTCACC
CAGGAAAAGGTCCCATGGGGCATCGTGCTCCTGCTGGGCGGAGGTTTCGCCCTG
GCTAAGGGCAGCGAAGCCAGCGGTCTGTCCGTGTGGATGGGAAAACAGATGGAG
CCGCTGCACGCTGTGCCCCCTGCTGCCATCACTCTCATTCTGTCCCTGCTTGTCGC
CGTGTTTACCGAGTGCACCTCCAACGTCGCTACCACTACTCTGTTCTTGCCCATCT
TCGCCTCGATGTCGAGATCCATCGGCCTGAACCCCCTCTACATTATGCTGCCGTG
CACCTTGTCCGCCTCCTTCGCGTTCATGCTGCCTGTGGCGACTCCCCCGAATGCTA
TTGTGTTCACCTACGGCCATCTCAAGGTCGCAGACATGGTCAAGACCGGAGTGAT
CATGAACATTATTGGCGTGTTCTGCGTGTTCTTGGCCGTGAATACCTGGGGTAGA
GCAATTTTCGACCTTGACCACTTCCCGGACTGGGCCAACGTGACCCATATCGAAA
CT

Another aspect of the invention relates to an expression cassette comprising a polynucleotide comprising a human SLC13A5 open reading frame. In certain embodiments, the polynucleotide is a human codon-optimized sequence, e.g., a polynucleotide comprising the nucleotide sequence of SEQ ID NO:2, or a sequence at least about 70% identical thereto, e.g., at least about 70, 75, 80, 85, 90, 91, 92, 93, 94, 95, 96, 97, 98, or 99% identical thereto.

The SLC13A5 open reading frame in the expression cassette may be operably linked to one or more expression elements that may enhance expression of SLC13A5 and/or Na⁺/citrate cotransporter. In some embodiments, the polynucleotide may be operably linked to a promoter. In some embodiments, a promoter of the present invention may be a ubiquitous promoter, e.g., a UsP promoter, e.g., as described in Karumuthil-Melethil et al. 2016 Hum. Gene Ther. (7):509-521. In some embodiments, the polynucleotide is operably linked to a promoter, e.g., a UsP promoter, e.g., a promoter comprising, consisting essentially of, or consisting of the nucleotide sequence of SEQ ID NO:3 or a sequence at least about 70% identical thereto, e.g., at least about 70, 75, 80, 85, 90, 91, 92, 93, 94, 95, 96, 97, 98, or 99% identical thereto.

SEQ ID NO:3. UsP promoter
GGGCGGAGTTAGGGCGGAGCCAATCAGCGTGCGCCGTTCCGAAAGTTGCCTTTT
ATGGCTGGGCGGAGAATGGGCGGTGAACGCCGATGATTATATAAGGACGCGCCG
GGTGTGGCACAGCTAGTTCCGTCGCAGCCGGGATTTGGGTCGCGGTTCTTGTTTG
TGGATCCCTGTGATCGTCACTTGGTAAGTCACTGACTGTCTATGCCTGGGAAAGG
GTGGGCAGGAGATGGGGCAGTGCAGGAAAAGTGGCACTATGAACCCTGCAGCCC
TAGGAATGCATCTAGACAATTGTACTAACCTTCTTCTCTTTCCTCTCCTGACAG

In some embodiments, the SLC13A5 open reading frame is operably linked to a polyadenylation signal, e.g., a synthetic polyadenylation signal, e.g., a polyadenylation signal comprising, consisting essentially of, or consisting of the nucleotide sequence of SEQ ID NO:4 or a sequence at least about 70% identical thereto, e.g., at least about 70, 75, 80, 85, 90, 91, 92, 93, 94, 95, 96, 97, 98, or 99% identical thereto.
**SEQ ID NO:4.** Synthetic polyadenylation signal (SpA)
AATAAAGAGCTCAGATGCATCGATCAGAGTGTGTTGGTTTTTTGTGTG

Those skilled in the art will further appreciate that a variety of promoter/enhancer elements may be used depending on the level and tissue-specific expression desired. The promoter/enhancer may be constitutive or inducible, depending on the pattern of expression desired. The promoter/enhancer may be native or foreign and can be a natural or a synthetic sequence. By foreign, it is intended that the transcriptional initiation region is not found in the wild-type host into which the transcriptional initiation region is introduced.

Promoter/enhancer elements can be native to the target cell or subject to be treated and/or native to the heterologous nucleic acid sequence. The promoter/enhancer element is generally chosen so that it will function in the target cell(s) of interest. In representative embodiments, the promoter/enhancer element is a mammalian promoter/enhancer element. The promoter/enhance element may be constitutive or inducible.

Inducible expression control elements are generally used in those applications in which it is desirable to provide regulation over expression of the heterologous nucleic acid sequence(s). Inducible promoters/enhancer elements for gene delivery can be tissue-specific or tissue-preferred promoter/enhancer elements, and include muscle specific or preferred (including cardiac, skeletal and/or smooth muscle), neural tissue specific or preferred (including brain-specific), eye (including retina-specific and cornea-specific), liver specific or preferred, bone marrow specific or preferred, pancreatic specific or preferred, spleen specific or preferred, and lung specific or preferred promoter/enhancer elements. Other inducible promoter/enhancer elements include hormone-inducible and metal-inducible elements. Exemplary inducible promoters/enhancer elements include, but are not limited to, a Tet on/off element, a RU486-inducible promoter, an ecdysone-inducible promoter, a rapamycin-inducible promoter, and a metallothionein promoter.

In embodiments wherein the SLC13A5 open reading frame is transcribed and then translated in the target cells, specific initiation signals are generally employed for efficient translation of inserted protein coding sequences. These exogenous translational control sequences, which may include the ATG initiation codon (i.e., translation start site) and adjacent sequences, can be of a variety of origins, both natural and synthetic.

In certain embodiments, the expression cassette further comprises at least one adeno-associated virus (AAV) inverted terminal repeat (ITR), e.g., two AAV ITRs. The two ITRs may have the same nucleotide sequence or different nucleotide sequences. The AAV ITRs may be from any AAV serotype, e.g., AAV2. Each ITR independently may be the wild-type sequence or a modified sequence. In some embodiments, a modified ITR may have a D-element deletion (WO 01/92551). A D-element deletion is defined as the removal of that portion of the ITR known as the D-element. The D-element can be alternatively referred to or known as a D region, or D sequence, and/or the nucleotides of the ITR that do not form palindromic hairpin structures. In some embodiments, the expression cassette is an AAV genome, e.g., a self-complementary AAV genome.

In certain embodiments, the expression cassette comprises a promoter, a human SLC13A5 open reading frame, and a polyadenylation site, optionally in the recited order. In certain embodiments, the expression cassette comprises an AAV ITR, a promoter, a human SLC13A5 open reading frame, a polyadenylation site, and an AAV ITR, optionally in the recited order. In certain embodiments, the expression cassette comprises an UsP promoter, a human SLC13A5 open reading frame, and a SpA polyadenylation site, optionally in the recited order. In certain embodiments, the expression cassette comprises an AAV ITR, an UsP promoter, a human SLC13A5 open reading frame, a SpA polyadenylation site, and an AAV ITR, optionally in the recited order. In certain embodiments, the expression cassette comprises an AAV2 ITR, a UsP promoter, a human SLC13A5 open reading frame, a SpA polyadenylation site, and an AA2V ITR, optionally in the recited order. In certain embodiments, the expression cassette comprises a modified AAV2 ITR, a UsP promoter, a human SLC13A5 open reading frame, a SpA polyadenylation site, and a wildtype AAV2 ITR, optionally in the recited order. The aforementioned components are in operable linkage.

In some embodiments, the expression cassette comprise, consists essentially of, or consists of the nucleotide sequence of SEQ ID NO:5 or a sequence at least about 70% identical thereto, e.g., at least about 70, 75, 80, 85, 90, 91, 92, 93, 94, 95, 96, 97, 98, or 99% identical thereto.

SEQ ID NO:5. Human SLC13A5 expression cassette excluding ITRs
GGTTCGGTACCGGGCGGAGTTAGGGCGGAGCCAATCAGCGTGCGCCGTTCCGAA
AGTTGCCTTTTATGGCTGGGCGGAGAATGGGCGGTGAACGCCGATGATTATATAA
GGACGCGCCGGGTGTGGCACAGCTAGTTCCGTCGCAGCCGGGATTTGGGTCGCG
GTTCTTGTTTGTGGATCCCTGTGATCGTCACTTGGTAAGTCACTGACTGTCTATGC
CTGGGAAAGGGTGGGCAGGAGATGGGGCAGTGCAGGAAAAGTGGCACTATGAA
CCCTGCAGCCCTAGGAATGCATCTAGACAATTGTACTAACCTTCTTCTCTTTCCTC
TCCTGACAGTCCGGAACCGGTGCCACCATGGCCTCGGCGTTGAGCTACGTGTCCA
AGTTCAAGTCCTTTGTGATCCTGTTTGTGACCCCTCTCTTGCTCCTGCCACTCGTG
ATTCTGATGCCGGCCAAGTTTGTGCGCTGCGCCTACGTGATCATCCTGATGGCAA
TCTACTGGTGTACCGAAGTCATCCCCCTCGCCGTGACTTCTCTTATGCCGGTGCTG
CTGTTCCCGCTGTTCCAAATCCTCGACAGCCGCCAGGTCTGCGTGCAGTACATGA
AGGACACCAACATGCTGTTCCTGGGCGGCCTGATTGTGGCCGTGGCAGTGGAGC
GGTGGAACCTCCACAAGCGCATCGCCCTGAGGACTCTGCTCTGGGTCGGCGCTA
AGCCAGCGCGGCTGATGCTGGGGTTCATGGGAGTGACCGCGCTGCTCTCCATGTG
GATCAGCAACACCGCCACTACCGCCATGATGGTCCCGATCGTGGAAGCCATTCTG
CAACAGATGGAAGCTACTTCCGCCGCCACTGAAGCGGGACTGGAACTGGTGGAC
AAGGGAAAGGCCAAGGAACTGCCCGGTTCACAAGTCATCTTCGAGGGACCAACT
CTGGGCCAGCAGGAGGACCAAGAGCGCAAGCGGCTGTGCAAGGCCATGACCTTG
TGTATCTGCTACGCCGCTTCGATCGGAGGCACTGCCACCCTGACCGGGACTGGAC
CCAACGTCGTGCTGCTGGGACAGATGAACGAGCTGTTCCCGGATTCCAAGGACCT
TGTGAACTTCGCGTCGTGGTTCGCCTTCGCATTCCCTAACATGCTCGTGATGCTGC
TCTTTGCATGGCTGTGGCTGCAGTTCGTGTATATGCGGTTTAACTTCAAGAAGTCC
TGGGGATGCGGTCTGGAATCCAAGAAGAACGAGAAGGCCGCGCTTAAGGTCCTC
CAGGAAGAATACCGGAAATTGGGGCCGCTGAGCTTCGCCGAGATCAACGTGCTC
ATCTGTTTCTTCCTGCTGGTCATCCTGTGGTTCTCAAGAGATCCTGGTTTCATGCC
GGGGTGGCTCACTGTGGCCTGGGTCGAAGGAGAAACCAAATACGTGTCGGATGC
GACTGTGGCAATCTTTGTCGCCACGCTCCTGTTCATCGTGCCTTCCCAAAAGCCTA
AGTTCAACTTCCGCTCCCAAACCGAAGAGGAGCGCAAAACACCGTTTTACCCTCC
ACCTCTGTTGGACTGGAAAGTCACCCAGGAAAAGGTCCCATGGGGCATCGTGCT

CCTGCTGGGCGGAGGTTTCGCCCTGGCTAAGGGCAGCGAAGCCAGCGGTCTGTC
CGTGTGGATGGGAAAACAGATGGAGCCGCTGCACGCTGTGCCCCCTGCTGCCAT
CACTCTCATTCTGTCCCTGCTTGTCGCCGTGTTTACCGAGTGCACCTCCAACGTCG
CTACCACTACTCTGTTCTTGCCCATCTTCGCCTCGATGTCGAGATCCATCGGCCTG
AACCCCCTCTACATTATGCTGCCGTGCACCTTGTCCGCCTCCTTCGCGTTCATGCT
GCCTGTGGCGACTCCCCCGAATGCTATTGTGTTCACCTACGGCCATCTCAAGGTC
GCAGACATGGTCAAGACCGGAGTGATCATGAACATTATTGGCGTGTTCTGCGTGT
TCTTGGCCGTGAATACCTGGGGTAGAGCAATTTTCGACCTTGACCACTTCCCGGA
CTGGGCCAACGTGACCCATATCGAAACTTGATAGGCGGCCGCGGAGCTCTCGAG
AGGCCTAATAAAGAGCTCAGATGCATCGATCAGAGTGTGTTGGTTTTTTGTGTGA
CGCGT

A further aspect of the invention relates to a vector comprising the polynucleotide or the expression cassette of the invention. Suitable vectors include, but are not limited to, a plasmid, phage, viral vector (e.g., an AAV vector, a lentiviral vector, an adenovirus vector, a herpesvirus vector, an alphavirus vector, or a baculovirus vector), bacterial artificial chromosome (BAC), or yeast artificial chromosome (YAC). For example, the nucleic acid can comprise, consist of, or consist essentially of an AAV vector comprising a 5' and/or 3' ITR (e.g., 5' and/or 3' AAV ITR). In some embodiments, the vector is a delivery vehicle such as a particle (e.g., a microparticle or nanoparticle) or a liposome to which the expression cassette is attached or in which the expression cassette is embedded. The vector may be any delivery vehicle suitable to carry the expression cassette into a cell.

In some embodiments, the vector is a viral vector, e.g., a lentiviral vector and/or an AAV vector. The AAV vector may be any AAV serotype, e.g., AAV9. In some embodiments, the AAV vector may comprise wild-type capsid proteins. In other embodiments, the AAV vector may comprise a modified capsid protein with altered tropism compared to a wild-type capsid protein, e.g., a modified capsid protein that is liver-detargeted or has enhanced tropism for particular cells.

In some embodiments, the vector is a single-stranded AAV (ssAAV) vector. In some embodiments, the vector is a self-complementary or duplexed AAV (scAAV) vector. scAAV vectors are described in international patent publication WO 01/92551 (the disclosure of which is incorporated herein by reference in its entirety). Use of scAAV to express the SLC13A5 ORF may provide an increase in the number of cells transduced, the copy number per transduced cell, or both.

An additional aspect of the invention relates to a transformed cell comprising the polynucleotide, expression cassette, and/or vector of the invention. In some embodiments, the polynucleotide, expression cassette, and/or vector is stably incorporated into the cell genome. The cell may be an *in vitro, ex vivo,* or *in vivo* cell.

Another aspect of the invention relates to a non-human transgenic animal comprising the polynucleotide, expression cassette, vector, and/or the transformed cell of the invention. In some embodiments, the animal is a laboratory animal, e.g., a mouse, rat, rabbit, dog, monkey, or non-human primate.

A further aspect of the invention relates to a pharmaceutical formulation comprising the polynucleotide, expression cassette, vector, and/or transformed cell of the invention in a pharmaceutically acceptable carrier.

In a specific embodiment, the polynucleotide, expression cassette, vector, and/or transformed cell of the invention is isolated.

In another specific embodiment, the polynucleotide, expression cassette, vector, and/or transformed cell of the invention is purified.

### Methods of Producing Virus Vectors

The present invention further provides methods of producing virus vectors. In one particular embodiment, the present invention provides a method of producing a recombinant AAV particle, comprising providing to a cell permissive for AAV replication: (a) a recombinant AAV template comprising (i) the polynucleotide or expression cassette of the invention, and (ii) an ITR; (b) a polynucleotide comprising Rep coding sequences and Cap coding sequences; under conditions sufficient for the replication and packaging of the recombinant AAV template; whereby recombinant AAV particles are produced in the cell. Conditions sufficient for the replication and packaging of the recombinant AAV template can be, e.g., the presence of AAV sequences sufficient for replication of the AAV template and encapsidation into AAV capsids (e.g., AAV *rep* sequences and AAV *cap* sequences) and helper sequences from adenovirus and/or herpesvirus. In particular embodiments, the AAV template comprises two AAV ITR sequences, which are located 5' and 3' to the polynucleotide of the invention, although they need not be directly contiguous thereto.

In some embodiments, the recombinant AAV template comprises an ITR that is not resolved by Rep to make duplexed AAV vectors as described in international patent publication WO 01/92551.

The AAV template and AAV *rep* and *cap* sequences are provided under conditions such that virus vector comprising the AAV template packaged within the AAV capsid is produced in the cell. The method can further comprise the step of collecting the virus vector from the cell. The virus vector can be collected from the medium and/or by lysing the cells.

The cell can be a cell that is permissive for AAV viral replication. Any suitable cell known in the art may be employed. In particular embodiments, the cell is a mammalian cell (e.g., a primate or human cell). As another option, the cell can be a trans-complementing packaging cell line that provides functions deleted from a replication-defective helper virus, e.g., 293 cells or other E1a trans-complementing cells.

The AAV replication and capsid sequences may be provided by any method known in the art. Current protocols typically express the AAV *rep*/*cap* genes on a single plasmid. The AAV replication and packaging sequences need not be provided together, although it may be convenient to do so. The AAV *rep* and/or *cap* sequences may be provided by any viral or non-viral vector. For example, the *rep*/*cap* sequences may be provided by a hybrid adenovirus or herpesvirus vector (e.g., inserted into the E1a or E3 regions of a deleted adenovirus vector). EBV vectors may also be employed to express the AAV *cap* and *rep* genes. One advantage of this method is that EBV vectors are episomal, yet will maintain a high copy number throughout successive cell divisions (i.e., are stably integrated into the cell as extra-chromosomal elements, designated as an "EBV based nuclear episome," *see* Margolski, (1992) Curr. Top. Microbiol. Immun. 158:67).

As a further alternative, the *rep*/*cap* sequences may be stably incorporated into a cell.

Typically the AAV *rep*/*cap* sequences will not be flanked by the TRs, to prevent rescue and/or packaging of these sequences.

The AAV template can be provided to the cell using any method known in the art. For example, the template can be supplied by a non-viral (e.g., plasmid) or viral vector. In particular embodiments, the AAV template is supplied by a herpesvirus or adenovirus vector (e.g., inserted into the E1a or E3 regions of a deleted adenovirus). As another illustration, Palombo et al. 1998 J. Virology 72:5025, describes a baculovirus vector carrying a reporter gene flanked by the AAV TRs. EBV vectors may also be employed to deliver the template, as described above with respect to the *rep*/*cap* genes.

In another representative embodiment, the AAV template is provided by a replicating rAAV virus. In still other embodiments, an AAV provirus comprising the AAV template is stably integrated into the chromosome of the cell.

To enhance virus titers, helper virus functions (e.g., adenovirus or herpesvirus) that promote a productive AAV infection can be provided to the cell. Helper virus sequences necessary for AAV replication are known in the art. Typically, these sequences will be provided by a helper adenovirus or herpesvirus vector. Alternatively, the adenovirus or herpesvirus sequences can be provided by another non-viral or viral vector, e.g., as a non-infectious adenovirus miniplasmid that carries all of the helper genes that promote efficient AAV production as described by Ferrari et al. 1997 Nature Med. 3:1295, and U.S. Patent Nos. 6,040,183 and 6,093,570.

Further, the helper virus functions may be provided by a packaging cell with the helper sequences embedded in the chromosome or maintained as a stable extrachromosomal element. Generally, the helper virus sequences cannot be packaged into AAV virions, e.g., are not flanked by ITRs.

Those skilled in the art will appreciate that it may be advantageous to provide the AAV replication and capsid sequences and the helper virus sequences (e.g., adenovirus sequences) on a single helper construct. This helper construct may be a non-viral or viral construct. As one nonlimiting illustration, the helper construct can be a hybrid adenovirus or hybrid herpesvirus comprising the AAV *rep*/*cap* genes.

In one particular embodiment, the AAV *rep*/*cap* sequences and the adenovirus helper sequences are supplied by a single adenovirus helper vector. This vector can further comprise the AAV template. The AAV *rep*/*cap* sequences and/or the AAV template can be inserted into a deleted region (e.g., the E1a or E3 regions) of the adenovirus.

In a further embodiment, the AAV *rep*/*cap* sequences and the adenovirus helper sequences are supplied by a single adenovirus helper vector. According to this embodiment, the AAV template can be provided as a plasmid template.

In another illustrative embodiment, the AAV *rep*/*cap* sequences and adenovirus helper sequences are provided by a single adenovirus helper vector, and the AAV template is integrated into the cell as a provirus. Alternatively, the AAV template is provided by an EBV vector that is maintained within the cell as an extrachromosomal element (e.g., as an EBV based nuclear episome).

In a further exemplary embodiment, the AAV *rep*/*cap* sequences and adenovirus helper sequences are provided by a single adenovirus helper. The AAV template can be provided as a separate replicating viral vector. For example, the AAV template can be provided by an AAV particle or a second recombinant adenovirus particle.

According to the foregoing methods, the hybrid adenovirus vector typically comprises the adenovirus 5' and 3' *cis* sequences sufficient for adenovirus replication and packaging (i.e., the adenovirus terminal repeats and PAC sequence). The AAV *rep*/*cap* sequences and, if present, the AAV template are embedded in the adenovirus backbone and are flanked by the 5' and 3' *cis* sequences, so that these sequences may be packaged into adenovirus capsids. As described above, the adenovirus helper sequences and the AAV *rep*/*cap* sequences are generally not flanked by ITRs so that these sequences are not packaged into the AAV virions.

Zhang et al. 2001 Gene Ther. 18:704-12) describe a chimeric helper comprising both adenovirus and the AAV *rep* and *cap* genes.

Herpesvirus may also be used as a helper virus in AAV packaging methods. Hybrid herpesviruses encoding the AAV Rep protein(s) may advantageously facilitate scalable AAV vector production schemes. A hybrid herpes simplex virus type I (HSV-1) vector expressing the AAV-2 *rep* and *cap* genes has been described (Conway et al. 1999 Gene Ther. 6:986 and WO 00/17377).

As a further alternative, the virus vectors of the invention can be produced in insect cells using baculovirus vectors to deliver the *rep*/*cap* genes and AAV template as described, for example, by Urabe et al. 2002 Human Gene Ther. 13:1935-43.

AAV vector stocks free of contaminating helper virus may be obtained by any method known in the art. For example, AAV and helper virus may be readily differentiated based on size. AAV may also be separated away from helper virus based on affinity for a heparin substrate (Zolotukhin et al. 1999 Gene Therapy 6:973). Deleted replication-defective helper viruses can be used so that any contaminating helper virus is not replication competent. As a further alternative, an adenovirus helper lacking late gene expression may be employed, as only adenovirus early gene expression is required to mediate packaging of AAV. Adenovirus mutants defective for late gene expression are known in the art (e.g., ts100K and ts149 adenovirus mutants).

### Methods of Using SLC13A5 Vectors

The present invention also relates to methods for delivering a SLC13A5 ORF to a cell or a subject to increase production of SLC13A5 and/or Na⁺/citrate cotransporter, e.g., for therapeutic or research purposes *in vitro, ex vivo,* or *in vivo.* Thus, one aspect of the invention relates to a method of expressing a SLC13A5 open reading frame in a cell, comprising contacting the cell with the polynucleotide, expression cassette, and/or the vector of the invention, thereby expressing the SLC13A5 open reading frame in the cell. In some embodiments, the cell is an *in vitro* cell, an *ex vivo* cell, or an *in vivo* cell. Expression of the present invention *in vitro* may be beneficial for research purposes, e.g., to evaluate efficacy and/or safety, prior to expression *in vivo.*

Another aspect of the invention relates to a method of expressing a SLC13A5 open reading frame in a subject, comprising delivering to the subject the polynucleotide, expression cassette, vector, and/or transformed cell of the invention, thereby expressing the SLC13A5 open reading frame in the subject. In some embodiments, the subject is an animal model of a disorder associated with aberrant SLC13A5 gene expression.

A further aspect of the invention relates to a method of treating a disorder associated with aberrant expression of a SLC13A5 gene or aberrant activity of a SLC13A5 gene product (e.g., Na⁺/citrate cotransporter) in a subject in need thereof, comprising delivering to the subject a therapeutically effective amount of the polynucleotide, expression cassette, vector, and/or transformed cell of the invention, thereby treating the disorder associated with aberrant expression of the SLC13A5 gene or aberrant activity of a SLC13A5 gene product in the subject. The invention provides a method of treating a disorder associated with aberrant expression of a SLC13A5 gene or aberrant activity of a SLC13A5 gene product (e.g., Na⁺/citrate cotransporter) in a subject in need thereof, comprising administering to the subject a therapeutically effective amount of the polynucleotide, the expression cassette, vector, and/or transformed cell of the invention, such that the SLC13A5 open reading frame is expressed in the subject. In some embodiments, the disorder associated with expression of the SLC13A5 gene or gene product may be citrate transporter disorder, e.g., SLC13A5 deficiency.

The invention further provides a method of treating citrate transporter disorder (e.g., SLC13A5 deficiency) in a subject in need thereof, comprising administering to the subject a therapeutically effective amount of the polynucleotide, the expression cassette, vector, and/or transformed cell of the invention, such that the SLC13A5 open reading frame is expressed in the subject.

In certain embodiments, the polynucleotide, expression cassette, vector, and/or transformed cell is delivered to the subject, e.g., systemically (e.g., intravenously) or directly to the liver of the subject. In certain embodiments, the polynucleotide, expression cassette, vector, and/or transformed cell is delivered to the subject, e.g., systemically (e.g., intravenously) or directly to the central nervous system (e.g., to the cerebrospinal fluid by intrathecal or intraventricular injection) of the subject. In some embodiments, the polynucleotide, expression cassette, vector, and/or transformed cell is delivered intravenously. In some embodiments, the polynucleotide, expression cassette, vector, and/or transformed cell is delivered intrathecally.

Recombinant virus vectors according to the present invention find use in both veterinary and medical applications. Suitable subjects include both avians and mammals. The term "avian" as used herein includes, but is not limited to, chickens, ducks, geese, quail, turkeys, pheasant, parrots, parakeets. The term "mammal" as used herein includes, but is not limited to, humans, primates, non-human primates (e.g., monkeys and baboons), cattle, sheep, goats, pigs, horses, cats, dogs, rabbits, rodents (e.g., rats, mice, hamsters, and the like), etc. Human subjects include neonates, infants, juveniles, and adults. Optionally, the subject is "in need of" the methods of the present invention, e.g., because the subject has or is believed at risk for a disorder including those described herein or that would benefit from the delivery of a polynucleotide including those described herein. As a further option, the subject can be a laboratory animal and/or an animal model of disease. Preferably, the subject is a human.

In certain embodiments, the polynucleotide of the invention is administered to a subject in need thereof as early as possible in the life of the subject, e.g., as soon as the subject is diagnosed with aberrant SLC13A5 and/or Na⁺/citrate cotransporter expression or activity or any of the above-mentioned diseases or disorders. In some embodiments, the polynucleotide is administered to a newborn subject, e.g., after newborn screening has identified aberrant SLC13A5 and/or Na⁺/citrate cotransporter expression or activity. In some embodiments, the polynucleotide is administered to a subject prior to the age of 5 years, e.g., prior to 1, 2, 3, 4, or 5 years of age. In some embodiments, the polynucleotide is administered to a fetus *in utero,* e.g., after prenatal screening has identified aberrant SLC13A5 and/or Na⁺/citrate cotransporter expression or activity or the presence of one of the above-mentioned diseases or disorders. In some embodiments, the polynucleotide is administered to a subject as soon as the subject develops symptoms associated with aberrant SLC13A5 and/or Na⁺/citrate cotransporter expression or activity or is suspected or diagnosed as having aberrant SLC13A5 and/or Na⁺/citrate cotransporter expression or activity or one of the above-mentioned diseases or disorders. In some embodiments, the polynucleotide is administered to a subject before the subject develops symptoms associated with aberrant SLC13A5 and/or Na⁺/citrate cotransporter expression or activity or disease/disorder, e.g., a subject that is suspected or diagnosed as having aberrant SLC13A5 and/or Na⁺/citrate cotransporter expression or activity or one of the above-mentioned diseases or disorders but has not started to exhibit symptoms.

In particular embodiments, the present invention provides a pharmaceutical composition comprising a polynucleotide, expression cassette, vector, and/or transformed cell of the invention in a pharmaceutically acceptable carrier and, optionally, other medicinal agents, pharmaceutical agents, stabilizing agents, buffers, carriers, adjuvants, diluents, etc. For injection, the carrier will typically be a liquid. For other methods of administration, the carrier may be either solid or liquid. For inhalation administration, the carrier will be respirable, and will preferably be in solid or liquid particulate form. In some embodiments, a pharmaceutical carrier may be D-sorbitol (e.g., PBS 5% w/v D-sorbitol).

By "pharmaceutically acceptable" it is meant a material that is not toxic or otherwise undesirable, i.e., the material may be administered to a subject without causing any undesirable biological effects.

One aspect of the present invention is a method of transferring an SLC13A5 ORF to a cell *in vitro.* The polynucleotide, expression cassette, and/or vector of the invention may be introduced to the cells in the appropriate amount. The virus vector may be introduced to the cells at the appropriate multiplicity of infection according to standard transduction methods appropriate for the particular target cells. Titers of the virus vector or capsid to administer can vary, depending upon the target cell type and number, and the particular virus vector or capsid, and can be determined by those of skill in the art without undue experimentation. In particular embodiments, at least about 10³ infectious units, more preferably at least about 10⁵ infectious units are introduced to the cell.

The cell(s) into which the polynucleotide, expression cassette, and/or vector of the invention, e.g., virus vector, can be introduced may be of any type, including but not limited to neural cells (including cells of the peripheral and central nervous systems, in particular, brain cells such as neurons, oligodendrocytes, glial cells, astrocytes), lung cells, cells of the eye (including retinal cells, retinal pigment epithelium, and corneal cells), epithelial cells (e.g., gut and respiratory epithelial cells), skeletal muscle cells (including myoblasts, myotubes and myofibers), diaphragm muscle cells, dendritic cells, pancreatic cells (including islet cells), hepatic cells, a cell of the gastrointestinal tract (including smooth muscle cells, epithelial cells), heart cells (including cardiomyocytes), bone cells (e.g., bone marrow stem cells), hematopoietic stem cells, spleen cells, keratinocytes, fibroblasts, endothelial cells, prostate cells, joint cells (including, e.g., cartilage, meniscus, synovium and bone marrow), germ cells, and the like. Alternatively, the cell may be any progenitor cell. As a further alternative, the cell can be a stem cell (e.g., neural stem cell, liver stem cell). As still a further alternative, the cell may be a cancer or tumor cell. Moreover, the cells can be from any species of origin, as indicated above.

The polynucleotide, expression cassette, and/or vector of the invention, e.g., virus vector, may be introduced to cells *in vitro* for the purpose of administering the modified cell to a subject. In particular embodiments, the cells have been removed from a subject, the polynucleotide, expression cassette, and/or vector of the invention, e.g., virus vector, is introduced therein, and the cells are then replaced back into the subject. Methods of removing cells from subject for treatment *ex vivo,* followed by introduction back into the subject are known in the art *(see,* e.g., U.S. patent No. 5,399,346). Alternatively, the polynucleotide, expression cassette, and/or vector of the invention, e.g., virus vector, is introduced into cells from another subject, into cultured cells, or into cells from any other suitable source, and the cells are administered to a subject in need thereof.

Suitable cells for *ex vivo* gene therapy are as described above. Dosages of the cells to administer to a subject will vary upon the age, condition and species of the subject, the type of cell, the nucleic acid being expressed by the cell, the mode of administration, and the like. Typically, at least about 10² to about 10⁸ or about 10³ to about 10⁶ cells will be administered per dose in a pharmaceutically acceptable carrier. In particular embodiments, the cells transduced with the virus vector *ex vivo* are administered to the subject in an effective amount in combination with a pharmaceutical carrier.

A further aspect of the invention is a method of administering the polynucleotide, expression cassette, and/or vector of the invention, e.g., virus vector, to a subject. In particular embodiments, the method comprises a method of delivering an SLC13A5 ORF to an animal subject, the method comprising: administering an effective amount of a virus vector according to the invention to an animal subject. Administration of the virus vectors of the present invention to a human subject or an animal in need thereof can be by any means known in the art. Optionally, the virus vector is delivered in an effective dose in a pharmaceutically acceptable carrier.

Dosages of the virus vectors to be administered to a subject will depend upon the mode of administration, the disease or condition to be treated, the individual subject's condition, the particular virus vector, and the nucleic acid to be delivered, and can be determined in a routine manner. Exemplary doses for achieving therapeutic effects are virus titers of at least about 10², 10³, 10⁴, 10⁵, 10⁶, 10⁷, 10⁸, 10⁹, 10¹⁰, 10¹¹, 10¹², 10¹³, 10¹⁴, 10¹⁵, 10¹⁶ transducing units or more, e.g., about 10⁷, 10⁸, 10⁹, 10¹⁰, 10¹¹, 10¹², 10¹³, 10¹⁴, or 10¹⁵ transducing units, yet more preferably about 10¹⁰, 10¹¹, 10¹², 10¹³, 10¹⁴, or 10¹⁵ transducing units (TU). Doses and virus titer transducing units may be calculated as vector or viral genomes (vg), and/or vg/kg of the subject. For example, in some embodiments, a dose for achieving therapeutic effects of the present invention may comprise a polynucleotide, expression cassette, vector, and/or transformed cell of the present invention in an amount of about 10¹⁰, 10¹¹, or 10¹² vg per murine subject or an equivalent dose for a human subject, or about 1 x 10¹⁰, 2 x 10¹⁰, 3 x 10¹⁰, 4 x 10¹⁰, 5 x 10¹⁰, 6 x 10¹⁰, 7 x 10¹⁰, 8 x 10¹⁰, 9 x 10¹⁰, 1 x 10¹¹, 2 x 10¹¹, 3 x 10¹¹, 4 x 10¹¹, 5 x 10¹¹, 6 x 10¹¹, 7 x 10¹¹, 8 x 10¹¹, 9 x 10¹¹, 1 x 10¹², 2 x 10¹², 3 x 10¹², 4 x 10¹², 5 x 10¹², 6 x 10¹², 7 x 10¹², 8 x 10¹², 9 x 10¹², or 10 x 10¹² or any value or range therein per murine subject or an equivalent dose for a human subject, e.g., about 1 x 10¹⁰ to about 1 x 10¹², about 5 x 10¹⁰ to about 9 x 10¹¹, about 1 x 10¹¹ to about 5 x 10¹², or about 1 x 10¹⁰, about 2 x 10¹¹, about 5 x 10¹¹, about 8 x 10¹¹, about 1 x 10¹² per murine subject or an equivalent dose for a human subject.

In particular embodiments, more than one administration (e.g., two, three, four or more administrations) may be employed to achieve the desired level of gene expression over a period of various intervals, e.g., daily, weekly, monthly, yearly, *etc.*

Exemplary modes of administration include oral, rectal, transmucosal, topical, intranasal, inhalation (e.g., via an aerosol), buccal (e.g., sublingual), vaginal, intrathecal, intraocular, transdermal, *in utero* (or *in ovo*), parenteral (e.g., intravenous, subcutaneous, intradermal, intramuscular [including administration to skeletal, diaphragm and/or cardiac muscle], intradermal, intrapleural, intracerebral, and intraarticular), topical (e.g., to both skin and mucosal surfaces, including airway surfaces, and transdermal administration), intro-lymphatic, and the like, as well as direct tissue or organ injection (e.g., to liver, skeletal muscle, cardiac muscle, diaphragm muscle or brain). Administration can also be to a tumor (e.g., in or a near a tumor or a lymph node). The most suitable route in any given case will depend on the nature and severity of the condition being treated and on the nature of the particular vector that is being used. In some embodiments, more than one mode and/or route of administration may be utilized, for example, e.g., intraparenchymal administration and intracerebroventricular administration.

In some embodiments, the viral vector is administered to the CNS, the peripheral nervous system, or both. In some embodiments, the viral vector is administered directly to the CNS, e.g., the brain or the spinal cord. Direct administration can result in high specificity of transduction of CNS cells, e.g., wherein at least 80%, 85%, 90%, 95% or more of the transduced cells are CNS cells. Any method known in the art to administer vectors directly to the CNS can be used. The vector may be introduced into the spinal cord, brainstem (medulla oblongata, pons), midbrain (hypothalamus, thalamus, epithalamus, pituitary gland, substantia nigra, pineal gland), cerebellum, telencephalon (corpus striatum, cerebrum including the occipital, temporal, parietal and frontal lobes, cortex, basal ganglia, hippocampus and amygdala), limbic system, neocortex, corpus striatum, cerebrum, and inferior colliculus. The vector may also be administered to different regions of the eye such as the retina, cornea or optic nerve. The vector may be delivered into the cerebrospinal fluid (e.g., by lumbar puncture) for more disperse administration of the vector.

The delivery vector may be administered to the desired region(s) of the CNS by any route known in the art, including but not limited to, intrathecal, intracerebral, intraventricular, intraparenchymal, intranasal, intra-aural, intra-ocular (e.g., intra-vitreous, sub-retinal, anterior chamber) and peri-ocular (e.g., sub-Tenon's region) delivery or any combination thereof.

The delivery vector may be administered in a manner that produces a more widespread, diffuse transduction of tissues, including the CNS, the peripheral nervous system, and/or other tissues.

Typically, the viral vector will be administered in a liquid formulation by direct injection (e.g., stereotactic injection) to the desired region or compartment in the CNS and/or other tissues. In some embodiments, the vector can be delivered via a reservoir and/or pump. In other embodiments, the vector may be provided by topical application to the desired region or by intra-nasal administration of an aerosol formulation. Administration to the eye or into the ear, may be by topical application of liquid droplets. As a further alternative, the vector may be administered as a solid, slow-release formulation. Controlled release of parvovirus and AAV vectors is described by international patent publication WO 01/91803.

Injectables can be prepared in conventional forms, either as liquid solutions or suspensions, solid forms suitable for solution or suspension in liquid prior to injection, or as emulsions. Alternatively, one may administer the virus vector in a local rather than systemic manner, for example, in a depot or sustained-release formulation. Further, the virus vector can be delivered dried to a surgically implantable matrix such as a bone graft substitute, a suture, a stent, and the like (e.g., as described in U.S. Patent 7,201,898).

Pharmaceutical compositions suitable for oral administration can be presented in discrete units, such as capsules, cachets, lozenges, or tablets, each containing a predetermined amount of the composition of this invention; as a powder or granules; as a solution or a suspension in an aqueous or non-aqueous liquid; or as an oil-in-water or water-in-oil emulsion. Oral delivery can be performed by complexing a virus vector of the present invention to a carrier capable of withstanding degradation by digestive enzymes in the gut of an animal. Examples of such carriers include plastic capsules or tablets, as known in the art. Such formulations are prepared by any suitable method of pharmacy, which includes the step of bringing into association the composition and a suitable carrier (which may contain one or more accessory ingredients as noted above). In general, the pharmaceutical composition according to embodiments of the present invention are prepared by uniformly and intimately admixing the composition with a liquid or finely divided solid carrier, or both, and then, if necessary, shaping the resulting mixture. For example, a tablet can be prepared by compressing or molding a powder or granules containing the composition, optionally with one or more accessory ingredients. Compressed tablets are prepared by compressing, in a suitable machine, the composition in a free-flowing form, such as a powder or granules optionally mixed with a binder, lubricant, inert diluent, and/or surface active/dispersing agent(s). Molded tablets are made by molding, in a suitable machine, the powdered compound moistened with an inert liquid binder.

Pharmaceutical compositions suitable for buccal (sub-lingual) administration include lozenges comprising the composition of this invention in a flavored base, usually sucrose and acacia or tragacanth; and pastilles comprising the composition in an inert base such as gelatin and glycerin or sucrose and acacia.

Pharmaceutical compositions suitable for parenteral administration can comprise sterile aqueous and non-aqueous injection solutions of the composition of this invention, which preparations are optionally isotonic with the blood of the intended recipient. These preparations can contain anti-oxidants, buffers, bacteriostats and solutes, which render the composition isotonic with the blood of the intended recipient. Aqueous and non-aqueous sterile suspensions, solutions and emulsions can include suspending agents and thickening agents. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

The compositions can be presented in unit/dose or multi-dose containers, for example, in sealed ampoules and vials, and can be stored in a freeze-dried (lyophilized) condition requiring only the addition of the sterile liquid carrier, for example, saline or water-for-injection immediately prior to use.

Extemporaneous injection solutions and suspensions can be prepared from sterile powders, granules and tablets of the kind previously described. For example, an injectable, stable, sterile composition of this invention in a unit dosage form in a sealed container can be provided. The composition can be provided in the form of a lyophilizate, which can be reconstituted with a suitable pharmaceutically acceptable carrier to form a liquid composition suitable for injection into a subject. The unit dosage form can be from about 1 µg to about 10 grams of the composition of this invention, or any value or range therein, e.g., from about 1 µg to about 5 grams, about 2 µg to about 10 µg, about 1.5 µg to about 7.5 grams, or about 1 µg, about 2 µg, about 3 µg, about 4 µg, about 5 µg, about 6 µg, about 7 µg, about 8 µg, about 9 µg, about 10 µg, about 20 µg, about 50 µg, about 100 µg, about 250 µg, about 500 µg, about 750 µg, about 1 gram, about 1.25 grams, about 1.5 grams, about 1.75 grams, about 2 grams, about 3 grams, about 4 grams, about 5 grams, about 6 grams, about 7 grams, about 8 grams, about 9 grams, or about 10 grams. When the composition is substantially water-insoluble, a sufficient amount of emulsifying agent, which is physiologically acceptable, can be included in sufficient quantity to emulsify the composition in an aqueous carrier. One such useful emulsifying agent is phosphatidyl choline.

Pharmaceutical compositions suitable for rectal administration can be presented as unit dose suppositories. These can be prepared by admixing the composition with one or more conventional solid carriers, such as for example, cocoa butter and then shaping the resulting mixture.

Pharmaceutical compositions of this invention suitable for topical application to the skin can take the form of an ointment, cream, lotion, paste, gel, spray, aerosol, or oil. Carriers that can be used include, but are not limited to, petroleum jelly, lanoline, polyethylene glycols, alcohols, transdermal enhancers, and combinations of two or more thereof. In some embodiments, for example, topical delivery can be performed by mixing a pharmaceutical composition of the present invention with a lipophilic reagent (e.g., DMSO) that is capable of passing into the skin.

Pharmaceutical compositions suitable for transdermal administration can be in the form of discrete patches adapted to remain in intimate contact with the epidermis of the subject for a prolonged period of time. Compositions suitable for transdermal administration can also be delivered by iontophoresis (*see,* for example, Pharm. Res. 3:318 (1986)) and typically take the form of an optionally buffered aqueous solution of the composition of this invention. Suitable formulations can comprise citrate or bis\tris buffer (pH 6) or ethanol/water and can contain from 0.1 to 0.2M active ingredient.

The virus vectors disclosed herein may be administered to the lungs of a subject by any suitable means, for example, by administering an aerosol suspension of respirable particles comprised of the virus vectors, which the subject inhales. The respirable particles may be liquid or solid. Aerosols of liquid particles comprising the virus vectors may be produced by any suitable means, such as with a pressure-driven aerosol nebulizer or an ultrasonic nebulizer, as is known to those of skill in the art. *See,* e.g., U.S. Patent No. 4,501,729. Aerosols of solid particles comprising the virus vectors may likewise be produced with any solid particulate medicament aerosol generator, by techniques known in the pharmaceutical art.

Having described the present invention, the same will be explained in greater detail in the following examples, which are included herein for illustration purposes only, and which are not intended to be limiting to the invention.

### EXAMPLES

### EXAMPLE 1:

A construct for gene therapy for SLC13A5 was generated using a ubiquitous UsP promoter (also referred to herein as pUSP), a 1704 nucleotide codon-optimized human SLC13A5 open reading frame (hSLC13A5opt), and a synthetic polyadenylation A tail **(****FIG. 1****).** While not wishing to be bound to theory, it is believed that a maximum number of cells should be transduced for improved therapeutic effectiveness, rather than the use of a promoter which may achieve the highest possible expression level per transduced cell. Therefore, a moderate to weak ubiquitous promoter, e.g., UsP promoter, was used. The construct was inserted into an AAV virus vector comprising a WT AAV2 ITR, an AAV2 ITR with a D-element deletion, and an AAV9 capsid.

The vector was tested for expression in HEK293T cells, examining mRNA, protein, and location. **FIG. 2** shows vector protein expression in HEK293T cells. Confocal microscopy of cells transfected with no vector (vehicle, **FIG. 2** top panel) or pUSP-hSLC13A5opt (hSLC13A5opt, **FIG. 2** bottom panel) 48 hours post-transfection staining with an antibody against human SLC13A5 showed no signal in vehicle treated cells while hSLC13A5opt transfected cells had a punctate pattern surrounding the cell.

Localization was found in the cell plasma membrane, as shown in **FIG 3****.** Confocal microscopy of a HEK293T cell transfected with pCbh-GFP and pUSP-hSLC13A5opt showed that 48 hours after transfection, GFP fluorescence was detected homogenously in the cytosol and did not co-localize with SLC13A5 staining (shown alone in **FIG. 3** top left panel; **FIG. 3** bottom panels, merged with GFP and DAPI), which was in a punctate pattern in the plasma membrane.

**FIGS. 4A-4C** show *in vivo* intrathecal administration of AAV9/USP-hSLC13A5opt reduced plasma citrate levels in SLC13A5 KO mice. Similarly to SLC13A5 patients, SLC13A5 KO mice have significantly increased baseline plasma citrate levels compared to WT mice. 1 month post-injection, WT mice **(****FIG. 4B****)** and KO mice **(****FIG. 4C****)** treated with vehicle **(****FIG. 4B****,** right bar; **FIG. 4C****,** middle bar) had equivalent plasma citrate levels compared to their respective baseline plasma levels, while KO mice treated with scAAV9/hSLC13A5ₒₚₜ **(****FIG. 4C****,** right bar) have significantly decreased plasma citrate levels.

**FIGS. 5A-5B** show that intrathecal AAV9/UsP-hSLC13A5opt treatment reduces EEG activity to normal levels in Slc13a5 KO mice. Slc13a5 knock-out (KO) mice and wild-type (WT) littermates were treated with vehicle or AAV9/UsP-hSLC13A5opt at ~ 3 months of age. At ~ 5 months post-injection, mice received wireless telemetry implants and EEG activity was recorded for two 60-hour sessions. **FIG. 5A** shows representative EEG tracings in WT and KO mice treated with vehicle or AAV9/UsP-hSLC13A5opt. Vehicle treated KO mice have increased epileptic discharges compared to WT mice or vector treated KO mice. **FIG. 5B** shows the quantification of epileptic spike trains. Vehicle treated KO mice **(****FIG. 5B****,** middle bar) have an increased number of spike trains as compared to vehicle treated WT mice **(****FIG. 5B****,** left bar) and AAV9/UsP-hSLC13A5opt treated KO mice **(FIG. 5C,** left bar).

**FIGS. 6A-6C** show that intrathecal AAV9/UsP-hSLC13A5opt treatment reduces seizure susceptibility and seizure-related death in Slc13a5 KO mice. Slc13a5 KO mice and WT littermates were treated with vehicle or AAV9/UsP-hSLC13A5opt at ~ 3 months of age. At ~ 6 months post-injection, following EEG recordings of baseline activity, mice received repetitive, low-dose (30 mg/kg) pentylenetetrazol (PTZ) injections and latency to convulsive seizures **(****FIG. 6A****)** and severity of seizures **(****FIG. 6B****)** were measured using the Racine Scale. **FIG. 6A** show vehicle treated KO mice have significantly shorter latency to seizure onset compared to vehicle treated WT littermates. Treatment with AAV9/UsP-hSLC13A5opt fully reduced the seizure latency to normal levels. **FIG. 6B** show that seizure severity is significantly increased in vehicle treated KO mice as compared to WT as shown by a higher Racine Score. Treatment with AAV9/UsP-hSLC13A5opt in KO mice restored seizure severity to WT levels. **FIG 6C** show that the increased seizure frequency and severity in KO mice was associated with a lower percentage of mice surviving PTZ injections as compared to WT mice. Treatment with AAV9/UsP-hSLC13A5opt in KO mice protected against seizure-related deaths and restored survival to WT levels.

All references cited herein are incorporated herein by reference in their entireties and for all purposes to the same extent as if each individual publication or patent or patent application was specifically and individually indicated to be incorporated by reference in its entirety for all purposes.

The foregoing examples are illustrative of the present invention, and are not to be construed as limiting thereof. Although the invention has been described in detail with reference to preferred embodiments, variations and modifications exist within the scope and spirit of the invention as described and defined in the following claims.
The following numbered clauses are not claims but represent preferred aspects and embodiments of the invention:

### Clauses

1. A polynucleotide comprising a human SLC13A5 open reading frame, wherein the human SLC13A5 open reading frame is codon-optimized for expression in a human cell.
2. The polynucleotide of clause 1, wherein the human SLC13A5 open reading frame comprises the nucleotide sequence of SEQ ID NO:2 or a nucleotide sequence having at least about 90% identity thereto.
3. An expression cassette comprising a polynucleotide comprising a human SLC13A5 open reading frame.
4. The expression cassette of clause 3, wherein the polynucleotide is the polynucleotide of clause 1 or 2.
5. The expression cassette of clause 3 or 4, wherein the human SLC13A5 open reading frame is operably linked to a promoter.
6. The expression cassette of clause 5, wherein the promoter is a UsP promoter.
7. The expression cassette of any one of clauses 3-6, wherein the human SLC13A5 open reading frame is operably linked to a polyadenylation signal.
8. The expression cassette of clause 7, wherein the polyadenylation signal is a synthetic polyadenylation signal.
9. The expression cassette of any one of clauses 3-8, further comprising at least one adeno-associated virus (AAV) inverted terminal repeat (ITR).
10. The expression cassette of clause 9, wherein the expression cassette comprises two AAV ITRs.
11. The expression cassette of clause 10, wherein the two AAV ITRs have the same nucleotide sequence.
12. The expression cassette of clause 10, wherein the two AAV ITRs have different nucleotide sequences.
13. The expression cassette of any one of clauses 10-12, wherein one of the two AAV ITRs is a modified ITR.
14. The expression cassette of any one of clauses 10-12, wherein one of the two AAV ITRs is a D-element deletion modified ITR.
15. The expression cassette of any one of clauses 9-14, wherein the AAV ITRs are AAV2 ITRs.
16. The expression cassette of any one of clauses 3-15, wherein the expression cassette is a self-complementary AAV genome.
17. The expression cassette of any one of clauses 3-16, wherein the expression cassette comprises a promoter, the human SLC13A5 open reading frame, and a polyadenylation site.
18. The expression cassette of any one of clauses 3-17, wherein the expression cassette comprises an AAV ITR, a promoter, the human SLC13A5 open reading frame, a polyadenylation site, and an AAV ITR.
19. The expression cassette of any one of clauses 3-18, wherein the expression cassette comprises an AAV ITR, a UsP promoter, the human SLC13A5 open reading frame, a synthetic polyadenylation site, and an AAV ITR.
20. The expression cassette of any one of clauses 3-19, wherein the expression cassette comprises an AAV ITR, a UsP promoter, the human SLC13A5 open reading frame, a synthetic polyadenylation site, and an AAV2 ITR.
21. The expression cassette of any one of clauses 3-20, wherein the expression cassette comprises a modified AAV2 ITR, a UsP promoter, the human SLC13A5 open reading frame, a synthetic polyadenylation site, and a wildtype AAV2 ITR.
22. The expression cassette of clause 20 or 21, comprising the nucleotide sequence of SEQ ID NO:5 or a sequence at least about 90% identical thereto.
23. A vector comprising the polynucleotide of clause 1 or 2 or the expression cassette of any one of clauses 3-22.
24. The vector of clause 23, wherein the vector is a viral vector.
25. The vector of clause 23, wherein the vector is an AAV vector.
26. The vector of clause 25, wherein the AAV vector is an AAV9 vector.
27. A transformed cell comprising the polynucleotide of clause 1 or 2, the expression cassette of any one of clauses 3-22, and/or the vector of any one of clauses 23-26.
28. The transformed cell of clause 27, wherein the polynucleotide, expression cassette, and/or vector is stably incorporated into the cell genome.
29. A non-human transgenic animal comprising the polynucleotide of clause 1 or 2, the expression cassette of any one of clauses 3-22, the vector of any one of clauses 23-26, and/or the transformed cell of clause 27 or 28.
30. A pharmaceutical composition comprising the polynucleotide of clause 1 or 2, the expression cassette of any one of clauses 3-22, the vector of any one of clauses 23-26, and/or the transformed cell of clause 27 or 28 in a pharmaceutically acceptable carrier.
31. A method of expressing a SLC13A5 open reading frame in a cell, comprising contacting the cell with the polynucleotide of clause 1 or 2, the expression cassette of any one of clauses 3-22, and/or the vector of any one of clauses 23-26, thereby expressing the SLC13A5 open reading frame in the cell.
32. A method of expressing a SLC13A5 open reading frame in a subject, comprising delivering to the subject the polynucleotide of clause 1 or 2, the expression cassette of any one of clauses 3-22, the vector of any one of clauses 23-26, and/or the transformed cell of clause 27 or 28, thereby expressing the SLC13A5 open reading frame in the subject.
33. A method of treating a disorder associated with aberrant expression of a SLC13A5 gene or aberrant activity of a SLC13A5 gene product in a subject in need thereof, comprising administering to the subject a therapeutically effective amount of the polynucleotide of clause 1 or 2, the expression cassette of any one of clauses 3-22, the vector of any one of clauses 23-26, and/or the transformed cell of clause 27 or 28, such that the SLC13A5 open reading frame is expressed in the subject.
34. The method of clause 33, wherein the disorder associated with expression of the SLC13A5 gene is citrate transporter disorder (e.g., SLC13A5 deficiency).
35. A method of treating citrate transporter disorder (e.g., SLC13A5 deficiency) in a subject in need thereof, comprising administering to the subject a therapeutically effective amount of the polynucleotide of clause 1 or 2, the expression cassette of any one of clauses 3-22, the vector of any one of clauses 23-26, and/or the transformed cell of clause 27 or 28, such that the SLC13A5 open reading frame is expressed in the subject.
36. The method of any one of clauses 32-35, wherein the subject exhibits symptoms of the disease prior to delivery of the polynucleotide, expression cassette, vector, and/or transformed cell.
37. The method of any one of clauses 32-36, wherein the polynucleotide, expression cassette, vector, and/or transformed cell is delivered *in utero.*
38. The method of any one of clauses 32-37, wherein the subject is a human.
39. The method of any one of clauses 32-38, wherein the polynucleotide, expression cassette, vector, and/or transformed cell is delivered to the nervous system of the subject.
40. The method of any one of clauses 32-38, wherein the polynucleotide, expression cassette, vector, and/or transformed cell is delivered to the liver of the subject.
41. The method of 32-40, wherein the polynucleotide, expression cassette, vector, and/or transformed cell is delivered intravenously.
42. The method of clause 39, wherein the polynucleotide, expression cassette, vector, and/or transformed cell is delivered by intrathecal, intracerebral, intraparenchymal, intracerebroventricular, intranasal, intra-aural, intra-ocular, or peri-ocular delivery, or any combination thereof.
43. The method of clause 42, wherein the polynucleotide, expression cassette, vector, and/or transformed cell is delivered intrathecally.
44. The method of clause 42, wherein the polynucleotide, expression cassette, vector, and/or transformed cell is delivered intracerebroventricularly.

## Claims

1. A polynucleotide comprising a human SLC13A5 open reading frame, wherein the human SLC13A5 open reading frame is codon-optimized for expression in a human cell.

2. The polynucleotide of claim 1, wherein the human SLC13A5 open reading frame comprises the nucleotide sequence of SEQ ID NO:2 or a nucleotide sequence having at least about 90% identity thereto.

3. An expression cassette comprising the polynucleotide of claim 1 or 2, optionally wherein the expression cassette is a self- complementary AAV genome.

4. The expression cassette of claim 3, wherein the human SLC13A5 open reading frame is operably linked to:
a) a promoter, optionally wherein the promoter is a UsP promoter; and/or
b) a polyadenylation signal, optionally wherein the polyadenylation signal is a synthetic polyadenylation signal.

5. The expression cassette of claim 3 or 4, further comprising at least one adeno-associated virus (AAV) inverted terminal repeat (ITR), optionally wherein the expression cassette comprises two AAV ITRs having the same or different nucleotide sequences, optionally wherein:
a) one of the two AAV ITRs is a modified ITR;
b) one of the two AAV ITRs is a D-element deletion modified ITR; and/or
c) at least one of the AAV ITRs is an AAV2 ITR.

6. The expression cassette of any one of claims 3-5, wherein the expression cassette comprises:
a) a promoter, the human SLC13A5 open reading frame, and a polyadenylation site;
b) an AAV ITR, a promoter, the human SLC13A5 open reading frame, a polyadenylation site, and an AAV ITR;
c) an AAV ITR, a UsP promoter, the human SLC13A5 open reading frame, a synthetic polyadenylation site, and an AAV ITR;
d) an AAV ITR, a UsP promoter, the human SLC13A5 open reading frame, a synthetic polyadenylation site, and an AAV2 ITR; or
e) a modified AAV2 ITR, a UsP promoter, the human SLC13A5 open reading frame, a synthetic polyadenylation site, and a wildtype AAV2 ITR,
optionally wherein the expression cassette of (d) or (e) comprises the nucleotide sequence of SEQ ID NO:5 or a sequence at least about 90% identical thereto.

7. A vector comprising the polynucleotide of claim 1 or 2 or the expression cassette of any one of claims 3-6, optionally wherein the vector is a viral vector or an AAV vector, optionally wherein the AAV vector is an AAV9 vector.

8. A transformed cell comprising the polynucleotide of claim 1 or 2, the expression cassette of any one of claims 3-6, and/or the vector of claim 7, optionally wherein the polynucleotide, expression cassette, and/or vector is stably incorporated into the cell genome.

9. A pharmaceutical composition comprising the polynucleotide of claim 1 or 2, the expression cassette of any one of claims 3-6, the vector of claim 7, and/or the transformed cell of claim 8 in a pharmaceutically acceptable carrier.

10. The polynucleotide of claim 1 or 2, the expression cassette of any one of claims 3-6, and/or the vector of claim 7 for use in a method of expressing a SLC13A5 open reading frame in a cell, the method comprising contacting the cell with the polynucleotide, the expression cassette, and/or the vector, thereby expressing the SLC13A5 open reading frame in the cell.

11. The polynucleotide of claim 1 or 2, the expression cassette of any one of claims 3-6, the vector of claim 7, the transformed cell of claim 8, and/or the pharmaceutical composition of claim 9 for use in a method of expressing a SLC13A5 open reading frame in a subject, the method comprising delivering the polynucleotide, the expression cassette, the vector, the transformed cell, and/or the pharmaceutical composition to the subject, thereby expressing the SLC13A5 open reading frame in the subject.

12. The polynucleotide of claim 1 or 2, the expression cassette of any one of claims 3-6, the vector of claim 7, the transformed cell of claim 8, and/or the pharmaceutical composition of claim 9 for use in a method of treating a disorder associated with aberrant expression of a SLC13A5 gene or aberrant activity of a SLC13A5 gene product in a subject in need thereof, the method comprising administering to the subject a therapeutically effective amount of the polynucleotide, the expression cassette, the vector, the transformed cell, and/or the pharmaceutical composition, such that the SLC13A5 open reading frame is expressed in the subject, optionally wherein the disorder associated with expression of the SLC13A5 gene is citrate transporter disorder, optionally wherein the disorder associated with expression of the SLC13A5 gene comprises SLC13A5 deficiency.

13. The polynucleotide of claim 1 or 2, the expression cassette of any one of claims 3-6, the vector of claim 7, the transformed cell of claim 8, and/or the pharmaceutical composition of claim 9 in a method of treating citrate transporter disorder (e.g., SLC13A5 deficiency) in a subject in need thereof, the method comprising administering to the subject a therapeutically effective amount of the polynucleotide, the expression cassette, the vector, the transformed cell, and/or the pharmaceutical composition such that the SLC13A5 open reading frame is expressed in the subject.

14. The composition for use of any one of claims 11-13, wherein the subject exhibits symptoms of the disease prior to delivery of the polynucleotide, expression cassette, vector, transformed cell, and/or pharmaceutical composition, and/or wherein the subject is a human.

15. The composition for use of any one of claims 11-14, wherein the polynucleotide, expression cassette, vector, transformed cell, and/or pharmaceutical composition is delivered in utero, to the nervous system, to the liver, and/or intravenously, optionally wherein delivery to the nervous system comprises intrathecal, intracerebral, intraparenchymal, intracerebroventricular, intranasal, intra-aural, intra-ocular, or peri-ocular delivery, or any combination thereof.
